## Europäisches Patentamt
## European Patent Office
## Office européen des brevets

(19)

(11) Publication number: **0 206 783**
**A2**

(12)

# EUROPEAN PATENT APPLICATION

(21) Application number: **86304764.3**

(22) Date of filing: **20.06.86**

(51) Int. Cl.⁴: **C 12 N 15/00,** C 12 P 21/02, C 12 N 1/18, C 07 K 7/10

(30) Priority: **20.06.85 US 747152**

(43) Date of publication of application: **30.12.86 Bulletin 86/52**

(84) Designated Contracting States: **AT BE CH DE FR GB IT LI LU NL SE**

(71) Applicant: **The Salk Institute Biotechnology Industrial Associates, Inc., P.O. Box 85200, San Diego California 92138 (US)**

(72) Inventor: **Thill, Gregory Patrick, 3720 Tomahawk Lane, San Diego California 92117 (US)**
Inventor: **Harpold, Michael Miller, 1341 29th Street, San Diego California (US)**
Inventor: **Tschopp, Juerb Friedrich, 12458 Carmei Cape, San Diego California (US)**

(74) Representative: **Bizley, Richard Edward et al, BOULT, WADE & TENNANT 27 Furnival Street, London EC4A 1PQ (GB)**

(54) **Expression and secretion of polypeptides from saccharomyces cerevisiae.**

(57) The invention provides improvements in alpha-factor leader peptide-facilitated secretion of polypeptides from S. cerevisiae into culture media. Recombinant expression vectors suitable for transformation of S. cerevisiae are provided which encode a fusion polypeptide including the pre-pro-alpha-factor leader peptide, or modification thereof, and the heterologous polypeptide to be secreted. Culture of S. cerevisiae strains transformed with an expression vector of the invention leads to secretion to the culture medium of heterologous polypeptide of interest. Increased recoveries of certain polypeptides, such as GRF, which retain their biological activities, are obtained by modifying peptide-encoding DNA sequences on the expression vector to produce analog polypeptides which are less subject to proteolytic degradation ·or other activity-reducing modifications during and after secretion. a-strains of S. cerevisiae are provided which, upon transformation with an expression vector according to the invention, with a fused gene controlled by the alpha-factor promoter, followed by culturing, secrete heterologous polypeptide in an alpha-factor-mediated secretion process. Peptide recovery is also enhanced by employing as host for transformation a S. cerevisiae strain with impaired proteolytic function.

## EXPRESSION AND SECRETION OF POLYPEPTIDES
## FROM SACCHAROMYCES CEREVISIAE

The present invention is directed to compositions and methods for improved expression of polypeptides in, and secretion of polypeptides from, genetically modified strains of Saccharomyces cerevisiae. More particularly, the invention is directed to recombinant vectors which provide improved efficiency in secretion of heterologous polypeptides, including large polypeptides of more than about 200 amino acids, from yeast cells, to yeast cells transformed with such vectors, and to methods of producing heterologous polypeptides which comprise culturing such transformed yeast cells.

There are recognized advantages to genetically modifying yeast strains for the purpose of producing desired polypeptides. Yeasts, like the prokaryotic cells that are commonly used as hosts for recombinant vectors, are rapidly proliferating and have the potential for producing large amounts of a particular protein. Furthermore, yeast cells are eukaryotic cells and frequently contain enzymes, lacking in prokaryates, for correctly processing heterologous fusion polypeptides, expressed from recombinant vectors, to produce a desired, biologically active form of a heterologous protein.

A significant limitation to the use of yeast strains as hosts for recombinant DNA vectors is the barrier that the cell wall of yeast cells presents to secretion of polypeptides to the culture medium. Purification in high yield of heterologous proteins that are expressed in yeast but not secreted through the cell wall into the culture medium is difficult and costly.

There exists a complex pathway for protein secretion from yeast cells, which includes the endoplasmic reticulum, the Golgi apparatus and secretory vesicles.

The ability of a polypeptide expressed in a yeast cell to traverse the yeast secretory pathway and be secreted through the cell wall into the culture medium depends on several factors. First, in the process of gene expression, a suitable leader peptide must be fused to the N-terminus of the polypeptide to be secreted. The first function of the leader is to permit the polypeptide to be secreted to enter the endoplasmic reticulum. Then the leader peptide-polypeptide fusion protein is glycosylated in several steps. Certain of these glycosylation steps, as well as aspects of the primary and three-dimensional structures of the fusion protein, serve as signals to direct the polypeptide to be secreted from the endoplasmic reticulum to the Golgi apparatus and from there to a secretory vesicle for secretion to the medium, rather than to some other destination, such as the periplasmic space, the plasma membrane, a lysosome, or a vacuole. At one or more points in the course of the secretory process, the leader peptide or parts thereof are proteolytically cleaved so that at least some of the polypeptide to be secreted is secreted to the medium without any amino acids from the leader remaining attached to the N-terminus.

The secretory process in yeast is so complex, and the effects on the process of altering the primary and three-dimensional structures of leader peptide-polypeptide fusion proteins so little understood, that there is considerable uncertainty in whether a particular heterologous fusion protein, involving a particular leader fused to a desired polypeptide to be secreted and expressed from a heterologous gene transformed into yeast, will result in improved yields of the desired polypeptide secreted to the medium.

As a means to effect secretion from Saccharomyces cerevisiae of recombinant DNA-encoded polypeptides, there has been considerable interest in the 89 amino acid leader peptide of the alpha-mating factor ("AMF") of Saccharomyces cerevisiae. AMF, also called "alpha-factor" is a short, 13 amino acid peptide that is secreted by alpha-strains of S. cerevisiae and has the sequence:

trp-his-trp-leu-gln-leu-lys-pro-gly-gln-pro-met-tyr.

This peptide functions as a pheromone, attracting a-strain S. cerevisiae yeast strains to promote mating between alpha and a-strains. It has been found that alpha-factor is initially expressed as a segment of a longer precursor protein, pre-pro-alpha-factor, which includes the leader peptide. The leader peptide, which is the amino terminal part of the pre-pro-alpha-factor, facilitates secretion of the four short alpha-factor peptide units in the pre-pro protein through the S. cerevisiae yeast secretory pathway.

It has been recognized that the alpha-factor leader peptide can be used to direct the secretion of other polypeptides. Discussion of alpha-factor, its precursor protein, and the use of the alpha-factor leader peptide for promoting secretion of other peptides are discussed for example in G. A. Bitter et al., Proc. Natl. Acad. Sci. USA 81, 5330-5334 (1984); A. Singh et al., Nucleic Acids Research 11, 4049-4063 (1983); J. Kurjan et al., Cell 30, 933-943 (1982); European Patent Application Publication Nos. 0 123 228, 0 123 294, 0 114 695, and 0 116 201. For example, European Patent Application Publication No. 0 123 228 describes the linkage of synthetic human insulin-like growth factor-I (IGF-I) and human insulin-like growth factor-II (IGF-II) genes to the alpha-factor leader peptide for the purpose of obtaining expression in, and secretion from, S. cerevisiae of IGF-I and IGF-II polypeptides.

In these schemes, the DNA sequences which encodes the alpha-factor leader peptide have been cleaved from the downstream DNA sequences which encode the alpha-factor peptide units, and the leader peptide-encoding sequence (or a portion thereof without coding sequence for certain amino acids in the processing site at the carboxy-terminus of the leader) has been combined with foreign DNA sequences that encode a foreign or heterologous polypeptide sought to be secreted from S. cerevisiae. In some cases, in which a yeast transformed with such a fused gene is cultured, expression and secretion to the culture medium of the polypeptide sought to be secreted is achieved. However, this technique is not universally successful. In many instances, secretion to the medium is at a very low level. In others, no secretion to the medium occurs at all, as the desired product, if expressed and not proteolytically degraded intracellularly, remains associated with the cell.

Significantly, it has been observed to be difficult to obtain, with alpha-factor leader, secretion of intact larger polypeptides, larger than about 100 amino acids. This may be due in part to failure of a larger polypeptide to be efficiently transported from organelle to organelle in the secretory pathway. Failure of mechanisms to cleave such a polypeptide from the alpha-factor leader peptide at appropriate points in the pathway, e.g., in the Golgi apparatus, may also result in a failure of the polypeptide to be secreted. Failure of a large, globular heterologous polypeptide, linked at its N-terminus to the alpha-factor leader, peptide, to be cleaved from the alpha-factor leader peptide may be due, at least in part, to steric interference to proteolytic processing enzyme action upon processing sites at the carboxy end of the leader and adjacent the amino-terminus of the large, globular heterologous polypeptide.

Even if some of a heterologous polypeptide is secreted into the culture medium from yeast cells, recovery of intact polypeptide with full biological activity may be limited by proteolytic degradation or other modifications of the polypeptide, which may occur prior to, during, or subsequent to the secretion process. For example, efficient secretion of beta-endorphin from S. cerevisiae yeast cells, in which the beta-endorphin-encoding DNA sequence is linked to the alpha-factor leader peptide-encoding DNA sequence, has been observed; however, recovery of full length beta-endorphin from the culture medium is very low due to proteolysis of the secreted peptide. Growth hormone releasing factor (GRF) has been similarly secreted from yeast cells transformed with a vector in which the leader peptide-encoding DNA sequence is linked to the GRF-encoding DNA sequence. However, both sulfoxidation of the methionine residues of the GRF polypeptide as well as proteolysis of the polypeptide are believed to limit recovery of full length peptide with full GRF activity.

The need remains for more efficient secretion of polypeptides from cultures of S. cerevisiae yeast cells into the culture medium and, in particular, improvement in alpha-factor facilitated release of polypeptides in biologically active form from the yeast cells into the medium. Furthermore, there exists a need for stabilizing polypeptides sought to be secreted from S. cerevisiae cells into the medium to prevent their loss of biological activity by proteolysis or other modifications.

The invention provides for improved secretion of a polypeptide of interest from S. cerevisiae yeast cells, that have been modified by transformation with a vector which includes a gene that is expressed in the cells and encodes a fusion polypeptide which consists of the polypeptide of interest fused, at its amino terminus, to the 89 amino acid alpha-factor leader peptide or a modification of said leader peptide.

In one aspect of the invention, it is found that the secretion to the culture medium of certain heterologous polypeptides, including larger, globular polypeptides, with more than about 200 amino acids, is enhanced by preparing an expression vector, such as a recombinant plasmid, in which there is a DNA sequence that encodes a fusion polypeptide in which a segment of one or two, but not three or four, of the alpha-factor peptide units (with intervening processing sites, if more than one unit) is left attached to the carboxy-terminus of the alpha-factor leader peptide and is attached, in turn, to the amino-terminus of the heterologous (foreign) polypeptide of interest. The DNA sequence encodes a processing site peptide fused to the amino-terminus of the segment of alpha-factor units and a processing site peptide between the segment of alpha-factor units and the heterologous peptide. The processing site between the leader peptide and the segment of alpha-factor units is usually the carboxy-terminal hexapeptide lys-arg-glu-ala-glu-ala of the 89 amino acid alpha-factor leader peptide. The processing site between the segment of alpha-factor units and the heterologous polypeptide of interest has any of a number of sequences, as described in greater detail below. Apparently, one reason (of perhaps several) for the improved secretion achieved with the fusion proteins coded by such vectors is as follows: By producing a precursor polypeptide in which one or two alpha-factor units spatially separate the leader peptide from the heterologous product polypeptide, the processing sites, at which proteolytic cleavage of product polypeptide from leader peptide and alpha-factor units must occur during the secretory process to have the heterologous polypeptide of desired sequence secreted to the medium, are made more accessible to proteolytic processing enzymes than they would be in the absence of the alpha-factor units. Consequently, proteolytic cleavage of the leader peptide and alpha-factor peptides proceeds more efficiently from the heterologous polypeptide than cleavage of leader peptide alone would and more correctly processed heterologous polypeptide is made available for secretion to the medium. There probably are other factors contributing to this aspect of the invention, besides the essentially steric effects just described, but these other factors are unclear.

In accordance with a particular aspect of the invention, human prourokinase (i.e., the high molecular weight, 412 amino acid form of human urokinase disclosed in Great Britain Published Patent Application No. 2,121,050) is produced in S. cerevisiae cells. Secretion of the prourokinase is promoted by encoding a fusion precursor polypeptide with a DNA sequence in which the urokinase-encoding nucleotide sequence is preceded by the leader peptide-encoding sequence (including the proteolytic processing site-encoding sequence at the 3'-end of the leader-peptide encoding sequence) and one or two alpha-factor peptide-encoding sequences, with a proteolytic processing site-encoding sequence following the alpha-factor peptide-encoding sequences.

Further provided in accordance with the invention are DNA-encoded, modified or analog heterologous peptides, particularly modified GRF, in which substantial biological activity is retained in the expressed and secreted protein by modification which reduce proteolytic degradation and other activity-reducing modifications of the protein.

Further in accordance with the invention, an a-strain (i.e., a mating type a or Mat a strain) of S. cerevisiae yeast is transformed with expression vectors which express, under control of alpha-factor gene promoter, fusion polypeptides which include an alpha-factor leader peptide and a heterologous peptide to be secreted. Surprisingly, expression and secretion of the heterologous polypeptides employing such expression vectors in the a-strain is equivalent to expression and secretion employing the vectors with alpha-strains (i.e., mating type alpha or Mat alpha strains).

The invention also provides improved recovery of polypeptide from S. cerevisiae yeast, through the use of S. cerevisiae strains which have been modified to destroy certain proteolytic functions, thereby reducing proteolysis of the heterologous polypeptide.

Finally, the invention entails methods of making a heterologous polypeptide by culturing a S. cerevisiae cell according to the invention, i.e., a S. cerevisiae cell that has been transformed with an expression vector according to the invention whereby the heterologous polypeptide is expressed as part of a fusion protein which includes, fused to the N-terminus of the heterologous polypeptide, the alpha-factor leader peptide or a modification thereof.

## BRIEF DESCRIPTION OF THE DRAWINGS

FIGURE 1 is a restriction map of the entire alpha-mating factor gene;

FIGURE 2 is a restriction map of a cloning vector designated YSV303;

FIGURE 3 is a restriction map of a cloning vector designated TRP203;

FIGURE 4 is a restriction map of a cloning vector designated TRP205;

FIGURE 5 is a restriction map of a cloning vector designated TRP209;

FIGURE 6 is a restriction map of a cloning vector designated TRP206;

FIGURE 7 is a restriction map of cloning vectors designated TRP210 and TRP211;

FIGURE 8 is a restriction map of a cloning vector designated TRP213;

FIGURE 9 is a restriction map of a cloning vector designated TRP215; and

FIGURE 10 is a map of pUK203, which encodes a fusion polypeptide that includes human prourokinase fused at its N-terminus to a modified alpha-factor leader peptide that includes an alpha-factor peptide.

In the present specification, "AMF" means "alpha mating factor"; "2u" means "2 micron"; "ul" means "microliter"; "uM" means "micromolar"; "ug" means "microgram"; temperatures are in degrees Celsius; "alpha-factor leader peptide" means the polypeptide consisting of the first 89 amino acids of the protein encoded by the S. cerevisiae alpha mating factor gene as reported by Kurjan et al., Cell 30, 933-943 (1982), including the proteolytic processing site consisting of the hexapeptide lys-arg-glu-ala-glu-ala at the carboxy-terminus of the 89 amino acids; "pre-alpha-factor segment" or "AMF pre-segment" means the first 83 amino acids of the alpha-factor leader peptide; "alpha-factor peptide" or "alpha-factor unit" means a 13 amino acid polypeptide with the same sequence as free AMF; and "secretion", unless otherwise qualified, means secretion through the cell wall into the extracellular medium.

The present invention provides improvements in the expression, secretion and recovery of recombinant DNA products from Saccharomyces cerevisiae. More particularly, the invention advances the usefulness of alpha-factor leader peptide-facilitated secretion of heterologous polypeptides from yeast, i.e., where the heterologous polypeptide is initially expressed as the carboxy-terminal portion of a fusion polypeptide wherein the alpha-factor leader or a modification thereof is the amino-terminal portion.

Among the improvements of the present invention, compositions and methods to accomplish secretion from S. cerevisiae of fully biologically active heterologous polypeptides not heretofore secreted from the yeast are provided. Recombinant DNA-encoded polypeptides, which are analogs of naturally occurring polypeptides, are produced and secreted. These analogs have high biological activities, including biological activities equal to or better than the biological activities of the corresponding naturally occurring polypeptides and are advantageous for production in S. cerevisiae because they are not completely inactivated, either by chemical reactions that alter their amino acid residues or by proteolysis during or subsequent to the secretion process.

The invention further provides alpha-factor leader peptide-facilitated secretion of desired polypeptides from a-strains of S. cerevisiae.

Further, the invention provides strains of S. cerevisiae which are impaired in their ability to proteolytically degrade proteins, particularly heterologous proteins, and which are advantageously transformed with expression vectors according to the invention to provide improved recovery of biologically active, heterologous protein secreted from the transformed strains as a result of alpha-factor leader peptide facilitated secretion.

In accordance with one aspect of the invention, expression vectors are provided for alpha-factor leader-mediated secretion of heterologous polypeptides which are much larger than the heterologous polypeptides, of fewer than about 100 amino acids, heretofore successfully secreted from S. cerevisiae in biologically active form. In order to obtain alpha-factor leader peptide-facilitated secretion of larger peptides, one or two alpha-factor peptides (including a proteolytic processing site between them if there are two) are included in the fusion polypeptide, expressed from the expression vector, between the alpha-factor leader (or modification thereof) and the heterologous product polypeptide. Thus, for example, recombinant vectors are prepared for genetically modifying S. cerevisiae and which encode a fusion precursor peptide having the general formula $NH_2$-alpha-factor pre-segment-(processing site)$_1$-(alpha-factor)$_n$-(processing site)$_2$-polypeptide of interest, where n=1 or 2, preferably n=1, wherein (processing site)$_1$ and (processing site)$_2$ can have the same or different amino acid sequences, and wherein, if n=2, there is another processing site (with sequence that may be the same as that of either (processing site)$_1$ or (processing site)$_2$ or may differ from those of both (processing site)$_1$ and (processing site)$_2$) between the two alpha-factor peptides. Heretofore, it has been generally believed in the art that alpha-factor leader-mediated secretion of heterologous polypeptide is best accomplished by elimination of the four alpha-factor (and processing sites between the first and second, second and third, and third and fourth) which follow the leader peptide units in the naturally occurring, 165 amino acid pre-pro-alpha-factor protein.

While it is not absolutely necessary to include an alpha-factor unit in the DNA-encoded fusion polypeptide for smaller peptides, e.g., under about 50 amino acid residues, sought to be secreted, for larger polypeptides, it appears that, without inclusion of at least one alpha-factor unit in the fusion polypeptide, the proteolytic processing enzymes which cleave the product polypeptide from the leader peptide are sterically hindered from acting upon the processing sites. Whatever the underlying cause(s) may be, we have found that, by including one or two alpha-factor units, which units to separate the leader (or modification thereof) from the polypeptide of interest, sought to be secreted, the processing sites flanking the alpha-factor unit or units are apparently made more accessible to proteolytic cleavage by processing enzymes as more correctly processed polypeptide of interest is secreted. On the other hand, it is found that secretion is lower if three or four intact alpha-factor units are left between the leader peptide (or modification thereof) and the polypeptide of interest.

The invention also provides for the production in, and secretion from, S. cerevisiae yeast of GRF analogs. The term "GRF analogs" is specifically used to designate biologically active proteins which are capable of stimulating the release of growth hormones from the pituitary and which are more resistant to inactivation under yeast fermentation conditions than are naturally occurring GRF molecules. Exemplary GRF analogs are represented by the following amino acid sequence:

$$H - R_1 - Ala - Asp - Ala - Ile - Phe - Thr -$$
$$R_8 - Ser - R_{10}- Arg - R_{12}- R_{13}- Leu -$$
$$Gly - Gln - Leu - R_{18}- Ala - Arg - Lys -$$
$$Leu - Leu - R_{24}- R_{25} -Ile - R_{27}- R_{28}-$$
$$Arg - Gln - Gln - Gly - Glu - R_{34}- Asn -$$
$$Glu - Glu - R_{38}- R_{39}- R_{40}- R_{41}- R_{42}-$$
$$R_{43}- R_{44}- OH$$

wherein $R_1$ is Tyr, Phe, Leu or His; $R_8$ is Asn or Ser; $R_{10}$ is Tyr or Phe; $R_{12}$ is Arg or Lys; $R_{13}$ is Ile or Val; $R_{18}$ is Ser or Tyr; $R_{24}$ is His or Gln;

$R_{25}$ is Glu or Asp; $R_{27}$ is Ala, Ile, Leu or Val; $R_{28}$ is Ser or Asn; $R_{34}$ is Ser, Ala or Arg; $R_{38}$ is Arg, Ser or Gln; $R_{39}$ is Arg or Gly; $R_{40}$ is Ala, Arg or Ser; $R_{41}$ is Arg or Lys; $R_{42}$ is Phe, Ala or Val; $R_{43}$ is Arg or Asn; and $R_{44}$ is a natural amino acid, excluding Cys or Met, wherein any or all of the residues between $R_{28}$ and $R_{44}$, inclusive, may be deleted.

The abbreviations used above refer to the naturally occurring amino acids, as follows:

| | | |
|---|---|---|
| ala | is | alanine |
| arg | is | arginine |
| asn | is | asparagine |
| asp | is | aspartic acid |
| cys | is | cysteine |
| glu | is | glutamic acid |
| gln | is | glutamine |
| gly | is | glycine |
| his | is | histidine |
| ile | is | isoleucine |
| leu | is | leucine |
| lys | is | lysine |
| met | is | methionine |
| phe | is | phenylalanine |
| ser | is | serine |
| tyr | is | tyrosine |
| val | is | valine |

In one modification, the GRF-encoding DNA sequence is modified so that the methionine residue at position 27 of the secreted GRF molecule has been replaced with alanine, isoleucine, leucine or valine. The resulting analog molecules have biological activity comparable to that of naturally occurring GRF, while sulfoxidation of the methionine residue, which may deactivate GRF, is avoided. Thus, biologically active GRF analogs are secreted and recovered in substantially improved yield relative to naturally occurring hGRF (human GRF).

In another specific modification of a DNA sequence encoding a hGRF analog, the sequence encoding the four C-terminal amino acid residues are deleted. This modification of the GRF molecule has little effect on GRF biological activity. Both modifications, i.e., replacement of methionine at the 27-position and shortening the peptide at its C-terminus, as well as other amino acid substitutions suggested by the above amino acid sequence and further shortening of the peptide at its C-terminus, may be combined in a single GRF-analog molecule. Presently preferred GRF analogs which retain substantially complete GRF biological activity include [Leu$^{27}$]-hGRF(1-40)-OH and [Leu$^{27}$]-hGRF(1-44)OH authentic, carboxy-terminal-amidated hGRF(1-44) has the amino acid sequence:

tyr-ala-asp-ala-ile-phe-thr-asn-ser-tyr-arg-lys-val-
leu-gly-gln-leu-ser-ala-arg-lys-leu-leu-gln-asp-ile-met
ser-arg-gln-gln-gly-glu-ser-asn-gln-glu-arg-gly-ala-arg
ala-arg-leu-NH$_2$.

Because some of the naturally occurring GRF peptides are amidated, and the heterologous gene-encoded GRF analogs described herein are of the free acid form, the free acid GRF analogs encoded by recombinant vectors according to the invention may be amidated _in vitro_ subsequent to secretion from _S. cerevisiae_ yeast. However, the yeast-produced analogs described herein have GRF-biological activity without amidation.

Various experimental protocols, including isolation and modification of genes, construction of expression vectors, transformation of expression vectors into yeast strains, culturing of the transformed yeast strains to obtain desired heterologous polypeptides, and development of host yeast strains will now be described in greater detail.

The alpha-factor genomic sequences were isolated by screening a library of yeast DNA sequences from _Saccharomyces cerevisiae_ strain AB320 (HO, ade2-1, lys2-1, trp5-2, leu2-1, canl-100, ura3-1, ural-1, met4-1). The genomic sequences of the library are carried as a collection of Sau3A partials cloned into the BamHI site of YEpl3. YEpl3 is a yeast-E. coli shuttle vector widely available to the skilled in the art that is capable of selection and replication in both yeast and E. coli. Strain YEpl3 can be obtained from ATCC, the American Type Culture Collection, Rockville, Maryland, U.S.A., under accession number 37115. When YEpl3 is transformed into E. coli, selection is provided by the ampicillin-resistance (beta-lactamase) gene from pBR322, while replication is accomplished using the origin of replication from pBR322. When YEpl3 is transformed into yeast, selection is provided by the S. cerevisiae LEU2 gene. The wild type LEU2 gene provides enzymatic activity necessary to complement leu2-deficient strains, transforming them to leucine prototrophy. Replication of YEpl3 in yeast derives from the origin of replication of the 2u plasmid (sometimes referred to herein as the "2u circle").

E. coli was transformed with YEpl3 by using competent MC1061 cells and a heat shock step. Basically, cells are rendered competent by growing them to mid-log (OD600=0.3) and incubating them for 30 minutes in cold (0°C) 50mM $CaCl_2$ at 1/2 the volume in which they were grown. They are centrifuged and resuspended in 1/50 the original volume of 10% glycerol, 50mM $CaCl_2$ and aliquoted into 500ul portions, stored in eppendorf tubes, quick frozen in liquid $N_2$ and stored at -70°C. To transform E. coli, an aliquot of competent MC1061 is thawed on ice. 100ul of cell suspension are added to the DNA (10 ng in this case) and allowed to sit at 0°C for 15 minutes. The cells are then placed at 37°C for 5 minutes, followed by a 23°C incubation for 5 minutes. At this point, the cells are ready for selection. The frequency of transformation is approximately $10^3$ transformants/ng of plasmid DNA.

Transformants are identified by ampicillin resistance. Ampicillin resistance is determined by spreading the cells directly onto 1.5% agar plates containing L-broth (1% Bacto-Tryptone, 0.5% yeast extract, 1% sodium chloride) and 50 ug/ml ampicillin. The plates are then incubated at 37°C. Colonies which form at 37°C after 16 hours are considered ampicillin-resistant. A collection of $10^4$ ampicillin-resistant colonies was obtained from the YEp13 library. Each plate was overlayed with 1 ml of L-broth containing 50 ug/ml ampicillin. The colonies were pooled, and the 5 ml volume was adjusted to 7% dimethyl sulfoxide (DMSO) and stored at -20°C.

Hybridization filters were prepared. The colonies were plated onto five plates (15 cm diameter) containing L-broth and 50 ug/ml ampicillin at a density of 4000 colonies per plate. Duplicate filters were prepared for hybridization as follows: A single 15 cm diameter nitrocellulose filter was placed over the plate and lifted up. This filter, which now contained the cells from the plate, served as the master. The first filter was placed in contact with the master, and the filters were marked in three places to orient them with respect to each other. A second filter was separately placed over the master in the same fashion. The three filters were then placed (cell side up) on a 15 cm plate of 1.5% agar containing L-broth and 50 ug/ml of ampicillin and grown for 18 hours at 37°C. The next day the duplicate filters, but not the master (which is stored at 4°C), were prepared for hybridization by placing them on stacks of Whatman filter paper soaked in 1.5M NaCl, 0.5M NaOH for 5 minutes and then submerging the filters in the same solution for 30 seconds. They were then submerged in 1M Tris-HCl, pH 8.0, 1.5M NaCl for 30 seconds. The filters are submerged in 2xSSC (0.3M NaCl, 0.03M Na citrate, pH 7.0) for 1 minute and allowed to air dry at room temperature for 20 minutes. They were then baked at 80°C for 2 hours in a vacuum oven, after which they were ready for hybridization.

The prepared filters were probed with a 21-base probe having the sequence CGCAGCATTCTTCGCATTAGC derived from the known sequence of alpha-factor (nucleotides 36-56 of the coding region) published by Kurjan et al., _supra_. This probe was labeled with $^{32}$P as follows: 100 ng of the oligonucleotide was incubated in a 50ul reaction containing 50mM Tris-HCl, pH 7.6, 10mM $MgCl_2$, 5mM dithiothreitol ("DTT"), 0.1mM spermidine, 0.1mM EDTA,100 uCi of gamma-$^{32}$P ATP (SA=7000 Ci/mmol) and 10 units of polynucleotide kinase at 37°C for 30 minutes. The unincorporated radionucleotides were removed by gel filtration over Sephadex G-50 in 10mM Tris-HCl, 1mM EDTA, and the labeled oligonucleotide was stored in 10mM Tris, 1mM EDTA.

Prehybridization was performed at 42°C in 6xSSPE (1xSSPE is 0.18M NaCl, 10mM $NaPO_4$ pH 7.0, 1mM EDTA) 10x Denhardt's (1x Denhardt's is 0.02% bovine serum albumin, 0.02% Ficoll, 0.02% polyvinylpyrollidone), 0.5% SDS for 3 hours. Hybridization was performed as above but with 10% dextran sulfate, $10^6$ cpm of probe per ml of hybridization solution, at 42°C for 18 hours. The filters were washed with 2xSSC and 0.5% SDS at room temperature, and then again at 42°C, and then exposed for 1 day at -70°C with one intensifying screen to Kodak X-AR5 X-ray film. Seven positive colonies were identified.

Regions of a plate showing strong hybridization signals with the probe in duplicate were isolated and streaked on a 1.5% LB agar plate (i.e., 1.5% agar containing LB medium (0.5% Bacto-tryptone, 0.5% yeast extract, 0.25% NaCl, pH adjusted to 7.5 with NaOH)) containing 50 ug/ml ampicillin. From this plate, single colonies were isolated and placed into wells of an 8x12 well microtiter dish containing 100ul liquid LB + 50 ug/ml ampicillin. After 18 h growth at 37°C, they were adjusted to 15% glycerol and stored at -70°C. A stamping device capable of transferring all colonies of an 8x12 well microtiter dish (96 wells) to a 15 cm filter grown on a 1.5% LB + 50 ug/ml ampicillin plate was used to transfer single colony isolates from each positive hybridization region to a nitrocellulose filter in duplicate. These colonies were grown for 18 hours at 37°C, and the filters were hybridized with the 21 base oligomer as described above. Four positive colonies were isolated in this manner and grown up in 2 ml liquid cultures of LB + 50 ug/ml ampicillin [LB-AMP(50)] at 37°C with agitation for 5-18 hours.

DNA was prepared from 1.5 ml of this culture by spinning the cells out and decanting the media. The cells were suspended in 100ul of 50mM glucose, 10mM EDTA, 25mM Tris-HCl, pH 8.0. They were allowed to sit on ice 5 minutes, after which 200ul of 0.2N NaOH, 1% SDS was added, and the mixture was incubated 5 minutes at 0°C. To this mixture was added 150ul of 3 M sodium acetate, pH 5.2. After 10 minutes at 0°C, the tube was centrifuged for 10 minutes in a microfuge. The pellet was removed with a toothpick, and 400ul of a 1:1 mixture of phenol chloroform (saturated with 10mM Tris pH7.5, 1mM EDTA) was added. The aqueous layer (less than 400 ul) was removed after centrifugation, and 800ul of 95% ethanol was added thereto. After incubation at -70°C for 20 minutes, the sample was spun for 5 minutes in a microfuge. The pellet was washed with 1ml of 95% ethanol, air dried and dissolved in 20-50ul of 10mM Tris-HCl, 1mM EDTA, 10 ug/ml Ribonuclease A.

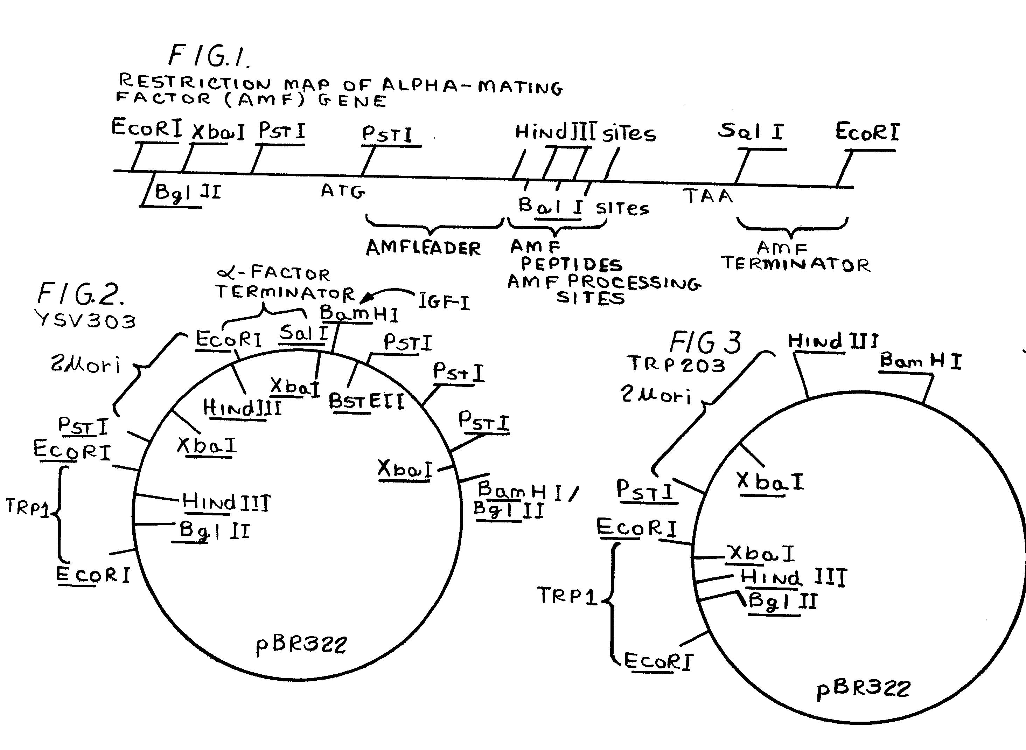

EcoRI restriction digests were performed by adding 1 ul of the mini-prepped DNA (200-500 ng) to the buffer prescribed by the manufacturer in a volume of 10 ul. The appropriate restriction enzyme was added at a concentration of 5-10 Units and the digest was incubated at 37°C for 1-2 hours. The digest was analyzed by agarose gel electrophoresis (0.8-1.2%), and fragment sizes were quantitated by reference to known

standards. Two colonies, AMF-B and AMF-D, had spectra similar to that published by Singh et al., supra. They both had the 1.7kb EcoRI fragment which should contain the promoter, structural gene and transcription terminator of alpha-factor (FIGURE 1). This fragment strongly hybridized to the oligonucleotide used as the probe in screening the library when the EcoRI digest was transferred to nitrocellulose and probed with the oligonucleotide, as directed below.

After electrophoresis, a photograph of the restriction digest and mobility markers (a HindIII digest of bacteriophage lambda DNA in this case) was taken in the presence of a ruler to measure the mobility of the fragments relative to the origin. The gel was then treated with 0.5N NaOH, 1.5M NaCl in a volume of 50 ml at room temperature with shaking for 30 minutes. It was subsequently neutralized by treatment with 1M Tris-HCl, pH 8.0, 1M NaCl in a volume of 50 ml at room temperature with shaking for 30 minutes. The gel was then placed on a stack of Whatman 3MM paper soaked in 20xSSC (3M NaCl, 0.3M Na citrate, pH 7.0). On top of the gel was placed a nitrocellulose filter which had been wetted in 2xSSC (0.3M NaCl, 0.03M Na citrate pH 7.0). The stack was topped with dry paper towels. The diffusion of the salt solution through the gel, past the nitrocellulose filter into the dry towels causes the transfer of DNA from the gel to the filter. The filter was then rinsed in 2xSSC and baked at 80°C for 2 hours. Hybridization was performed as follows: The filter was wetted in 5xSSPE [1xSSPE: 0.18M NaCl, 10mM $NaPO_4$ (pH 7.0), 1mM EDTA], 0.5% SDS, 1 mg/ml denatured, sheared, salmon sperm DNA. To this solution was added $5x10^5$ cpm/ml hybridization fluid of $^{32}P$-labeled oligonucleotide (the 21 base probe in this case). After hybridization at the specified temperature ($T_m$-5°C for oligonucleotide probes) for 12-18 hours, the filter was washed at room temperature in 2xSSC, 0.5% SDS in a volume of 200ml with agitation for 10 minutes.  This wash was repeated and the filter was washed in the same buffer but at the hybridization temperature with agitation for 10 minutes.  The filter was air dried and exposed to Kodak X-AR5 film with the presence of a DuPont intensifying screen at -70°C for 12 hours.

From the map and sequence generated by Kurjan et al., supra., it was predicted that most of the structural gene is present on a 500bp PstI-SalI fragment (FIGURE 1).  The genomic clone isolated from the YEpl3 library was digested with PstI and SalI and subjected to electrophoresis on 8% polyacrylamide.  The band was excised from the gel after visualization of the ethidium bromide-stained band via UV excitation.  The size of the band was determined via reference to molecular weight standards (a HaeIII digest of phiX174 phage DNA in this case).  This gel slice was placed in a dialysis bag containing electrophoresis buffer, lxTBE (0.089M Tris borate, 0.089M boric acid, 2mM EDTA) in this case.  The bag was placed in an electrophoresis chamber and run at 100 volts (constant voltage for 1-2 hr).  At the end of the run, the current was reversed for 1 minute.  The buffer, now containing the DNA, was removed from the dialysis bag, phenol-extracted and concentrated by extraction with secondary butyl alcohol, adjusted to 0.3M sodium acetate (NaOAc) and ethanol-precipitated.  The recovered DNA was quantitated via gel electrophoresis and referenced to standards of known concentration.

The PstI-SalI fragment was cloned into M13mp8 and M13mp9 vectors.  These vectors were cleaved with SalI and PstI, using manufacturer's specifications for restriction enzyme digestion, and diluted to 10 ng per microliter.  They were heated to 65°C to inactivate any remaining endonuclease activity.  In cases where restriction endonucleases are refractory to heat inactivation, the sample was phenol-extracted, ether-washed and ethanol-precipitated, then diluted to 10 ng/ul in TE. 10 ng of vectors and 50-100 ng of insert were ligated in the presence of 50mM Tris-HCl, pH 7.5, 10mM $MgCl_2$, 10mM dithiothrieitol, 1mM spermidine, 1mM ATP, and 200-400 Units (New England Biolabs) of T4 ligase. After a certain interval, 0.5-2 hours at 23°C or 5-16 hours at 16°C, the ligation reaction was used to transform E. coli JM103.

E. coli JM103 cells are rendered competent in the following fashion. They are seeded from a minimal plate and grown to OD550=0.3, centrifuged at 4°C and suspended in 1/2 volume of cold 50 mM $CaCl_2$, left on ice for 20 minutes and centrifuged. They are resuspended in 1/10 the original culture volume and stored at 4°C until used.

During transformation, 100-200 ul of culture of these cells were added to the ligation reaction mixture and placed on ice for 20 minutes. They were heated to 42°C for 2 minutes. Meanwhile, to a molten solution of 1% LB agar was added 30ul of a 20 mg/ml solution of X-gal⁻ (5 bromo-4 chloro-3-indoyl-beta-galactoside) in dimethyl formamide and 20ul of (24 mg/ml) isopropyl B-D-thiogalactoside. At the end of the two minute 42°C incubation, the cells and DNA were added to the top agar and spread on a room temperature 1.5% L-B plate. They were subsequently incubated at 37°C for 12-16 hours. Clones containing inserts appeared as white plaques, while reclosed or uncut vectors retained a blue color.

Plaques containing inserts of the PstI-SalI fragment were isolated in both M13mp8 and M13mp9 vectors in the manner described above. Template for sequencing was isolated in the following manner. A plaque was cored and placed in 1-2 ml of liquid LB and shaken for 5-6 hours at 37°C. A 1 ml aliquot of the supernatant was centrifuged and 900 ul was recovered. This was recentrifuged and 800 ul was recovered. 200 ul of 2.5M NaCl, 20% PEG6000 was added and the mixture was let stand for 15 minutes at 23°C. After centrifugation, the supernatant was completely removed and the pellet was resuspended in 100 ul of 10mM Tris-HCl, pH 7.5, 0.1mM EDTA (TE). 50 ul of TE-saturated phenol was added and the sample was extracted. Following centrifugation, the aqueous phase was extracted with diethyl ether and the aqueous phase was adjusted to 0.3M sodium acetate pH 5.2. 250 ul of ethanol was added and the solution was frozen in a dry ice-ethanol bath. Following centrifugation for 5 minutes, the pellet was resuspended in 50 ul TE.

5 ul aliquots were sequenced by the Sanger dideoxynucleotide method. This verified that the clone encoded alpha-factor sequences because they were, in fact, identical to the known published sequence, (Kurjan et al., supra.).

Subsequent to the cloning of alpha-factor by the methods of Kurjan et al. supra., Singh et al. supra. isolated two alpha-factor-like sequences. The first, MF1, is identical to that cloned by Kurjan et al., supra. The second, MF2, is somewhat different in that it contains coding segments for only two alpha-factor peptides, as opposed to the four in MF1. Additionally, one of these two peptides coded by MF2 is a variant of alpha-factor, in that it contains two conservative amino acid substitutions at positions 4 and 6. Although either of the alpha-factor genes could have been utilized in vector constructs described herein, it was chosen to use the EcoRI fragment characterized by Kurjan et al., supra., because it contains a HindIII restriction enzyme site at the junction of alpha-factor leader encoding segment and the segment encoding the first alpha-factor peptide unit, thereby, making cloning and manipulations easier.

The genomic clone, AMFB, containing the EcoRI-HindIII fragment, was digested with EcoRI and HindIII. The products were run on a 0.8% agarose gel in

1xTBE at 50V (constant voltage) overnight. A 1200 bp fragment was identified by reference to markers (a HaeIII digest of phiX174 phage DNA). Based upon the maps of Singh, et al. supra. and Kurjan et al. supra., this fragment was predicted to contain the promoter, and structural gene up to the segment encoding the first set of processing sites (at the carboxy-end of the leader), but to lack the alpha-factor unit-encoding sequence portions and 3'-non-translated region of the message. The fragment was electroeluted and ethanol-precipitated.

The vector, pBR322, was digested with EcoRI and HindIII. A ligation was set up using 40 ng of vector and 200 ng of the 1200 bp EcoRI-HindIII fragment in a 15 ul reaction volume at 15°C overnight. This ligation reaction was used to transform E. coli strain MC1061 to ampicillin resistance, as previously described. Eight mini preps were prepared, as described earlier, and digested with EcoRI and HindIII. Six of the eight transformants contained the 1200 bp fragment as an insert. This clone is referred to as p-alpha-factor. A large-scale DNA prep was prepared and extensively restriction mapped. The following enzymes were used according to manufacturer's specifications: BalI, BglII, HpaI, KpnI, SmaI, NruI, StuI, XbaI, XhoI, AvaI, BclI, BamHI, ClaI, EcoRV, PvuI, PvuII, SalI, SphI, AccI, HindIII.

As previously described, the DNA sequence of the 500bp PstI-SalI fragment was determined by Sanger dideoxy sequencing. The PstI site in question is located just inside the coding region, whereas the SalI site is located just beyond the translation termination codon.

The organization of the alpha-factor gene is graphically described in Kurjan et al., supra. Simply, there are 4 tandemly arranged segments, each coding for of the 13 amino acid alpha-factorpeptide, and with each 13 amino acid peptide preceded by the proteolytic processing site peptide with amino acid sequence lys-arg-glu-ala-glu-ala-glu-ala or a modification thereof. In the first processing site, which corresponds to the carboxy-terminal six amino acids of the leader, one of the glu-ala pairs is missing. In both the third and the fourth processing sites, aspartate replaces the second of the three glutamates.

The alpha-factor coding sequences terminate 36 bp upstream of a unique SalI site. To locate the 3'-end of the message transcribed from the alpha-factor gene, S1 nuclease mapping of the RNA was undertaken.

To map mRNA termini via S1 nuclease mapping, a restriction enzyme fragment (SalI-EcoRI 300bp fragment in this case) believed to have one end inside the segment transcribed into mRNA and one end outside was isolated via polyacrylamide gel electrophoresis on 8% acrylamide for 2.5h at 200V (constant voltage). This fragment was labeled on the 3'-end of the antisense strand so that when the antisense strand was hybridized to the alpha-factor mRNA the labeled nucleotide would be involved in hybrid formation (that is, protected). Labeling of 1 ug of this fragment was accomplished by filling in the 5'-overhang of the SalI site (i.e. labeling the 3'-end) with alpha-$^{32}$P dCTP in the presence of unlabeled TTP. The 40 ul reaction mixture contained 60mM NaCl, 10 mM Tris-HCl, pH 7.5, 10mM MgCl$_2$, 0.5mMTTP and 100 uCi alpha-$^{32}$P dCTP (2000-3000 Ci/mMol). The reaction was initiated by adding 5 Units of E. coli DNA polymerase (large fragment) and run for 20 minutes at 23°C. The labeled fragment was isolated from unincorporated radioactivity by column chromatography over Sephadex G-50. A total of 160,000 cpm was recovered.

RNA was isolated from alpha-cells as well as from a cells (as a control, as alpha-mating factor transcripts are specific to alpha-cells. In this case, X2180-1A (an a strain of S. cerevisiae) and 2102-alpha (an alpha strain of S. cerevisiae) were grown to mid-log and harvested by centrifugation, washed in $H_2O$, and washed once with RNA extraction buffer (REB) which is 0.1M LiCl, 0.1M Tris-HCl, pH 7.5, 0.1mM EDTA. The pellet was resuspended in an equal volume of REB and acid-washed glass beads (0.45 u diameter) were added to a volume equal to the original pellet. Cell breakage was accomplished by a series of 4x15 second bursts on a vortex mixer with a 15 second pause on ice between bursts. The mixture was then adjusted to 0.2% SDS and a volume of phenol/chloroform equal to the aqueous volume was added. Following extraction, the aqueous layer was removed and adjusted to 0.3M sodium acetate, pH 5.2. 2 volumes of ethanol were added and the RNA was precipitated at -20°C. The precipitated RNA was dissolved in $H_2O$ and stored at -20°C. Poly A+ RNA was isolated by the method described in T. Maniatis et al., <u>Cold Spring Harbor Laboratory Manual</u>, Cold Spring Harbor, New York (1982) (hereinafter CSH).

In the Sl mapping experiment, less than 20,000 cpm of 3'-labeled DNA was added to the following RNA samples 1) <u>a</u> strain poly A+, 10 ug, 2) <u>a</u> strain total RNA, 100 ug, 3) alpha-strain poly A+ RNA, 10 ug, 4) alpha-strain total RNA, 100 ug. The mixture was ethanol-precipitated and dissolved in 20 ul of hybridization buffer (80% formamide 0.4M NaCl, 0.04M Pipes, pH 6.4, 1mM EDTA) and heated to 70°C for 10 minutes, and allowed to hybridize overnight at 52°C. The hybridization reaction was added to 400 ul of Sl nuclease buffer (0.28M NaCl, 50 mM sodium acetate (pH 4.6), 4.5mM $ZnSO_4$) to which was added 10,000 Units of Sl nuclease (purchased from Boehringer Mannheim Biochemicals, Indianapolis, Indiana, U.S.A.). The sample was incubated at 40°C for 30 minutes. The sample was then phenol-extracted, carrier tRNA (5 ug) was added to the sample, and the mixture was ethanol-precipitated. It was dissolved in a sequencing gel buffer (80% formamide, 0.1% bromphenol blue, 0.1% xylene

cyanol and 1xTBE) and loaded onto an 8% polyacrylamide gel in 1xTBE containing 8 M urea. The four samples, along with the labeled restriction fragment and radiolabeled molecular weight markers, were run at 150 mA until the bromphenol blue reached the bottom. The film was then exposed to Kodak X-AR5 film with an intensifying screen for 6 hours at -70°C.

Specific hybrids of slightly lower molecular weight than the probes (50-100bp shorter) were located only in the alpha-strain-specific mRNA. The result was cleaner in the case of the poly A+ alpha-specific RNA, where two bands are clearly seen. The correct bands exist in alpha-specific total RNA, but there is a background of less intense bands in total RNA. The putative 3'-ends of alpha-factor mRNA were never seen with mRNA from the a-strain. Results showed that an alpha cell-specific transcript terminates in the SalI-EcoRI fragment just 5' of the EcoRI restriction site.

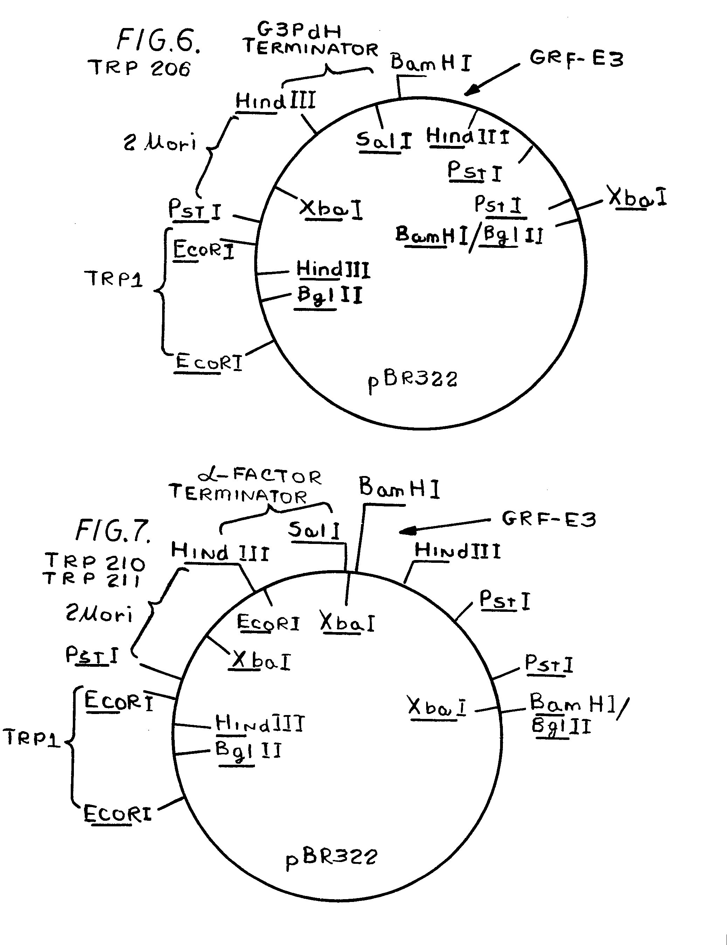

Alternative processing sites were constructed for certain vectors. Because enzymatic cleavage at lys-arg is more efficient than cleavage at glu-ala, two approaches were attempted and found to be successful. The first involves altering the position of the lys-arg residues relative to the glu-ala residues. Normally the lys-arg residues precede the glu-ala residues. A vector was constructed wherein, in the fusion protein encoded by the vector, the segment of glu-ala or asp-ala dipeptides of the processing site, nearest the amino-terminus of the heterologous polypeptide of interest to be secreted, were both preceded by and followed by the dipeptide lys-arg. Because the native gene codes for processing site peptide sequences which have glu-ala at their carboxy-termini and a HindIII site occurs in the gene segment encoding each of these carboxy-terminal glu-ala dipeptides, an oligonucleotide with a suitable HindIII sticky end can be used to insert a sequence encoding lys-arg between the sequence encoding the carboxy-terminal glu-ala of the processing site nearest the N-terminus of the polypeptide to be secreted and the sequence encoding the polypeptide to be secreted. Such constructs can be verified by restriction endonuclease digestion and DNA sequence analysis. An example of such a construct is TRP211 (FIGURE 7) or YSV303 (FIGURE 2). The constructions of TRP 211 and YSV303 are described hereinafter in greater detail.

Following the second approach, a vector can be constructed wherein the sequence encoding the amino acids other than lys-arg, e.g., glu-ala-glu-ala, in the processing site nearest the N-terminus of the heterologous peptide to be secreted are deleted so that, in the fusion protein, the peptide to be secreted is immediately preceded, at its N-terminus, by only the single dipeptide lys-arg as the processing site.

Such a vector also was constructed. The deletion was accomplished using a M13 mutagenesis protocol. The alpha-factor promoter, structural gene (including the segment encoding the 89-amino acid leader peptide with the hexapeptide lys-arg-gly-ala-glu-ala) (including the nucleotides to be deleted) and foreign genes were isolated via restriction digest of a plasmid YSV201 (described in greater detail hereinafter) and subcloned into an M13 vector digested with appropriate restriction enzymes. A single-stranded template was prepared, as for the M13 sequencing reactions. A primer homologous to the sequences on both sides of the region to be deleted was kinased and annealed to the template. Following extension and ligation, closed circular, double-stranded (replicative form) DNA was isolated on an alkaline sucrose gradient. These supercoiled molecules are dialyzed and used to transform competent E. coli JM103, as previously described.

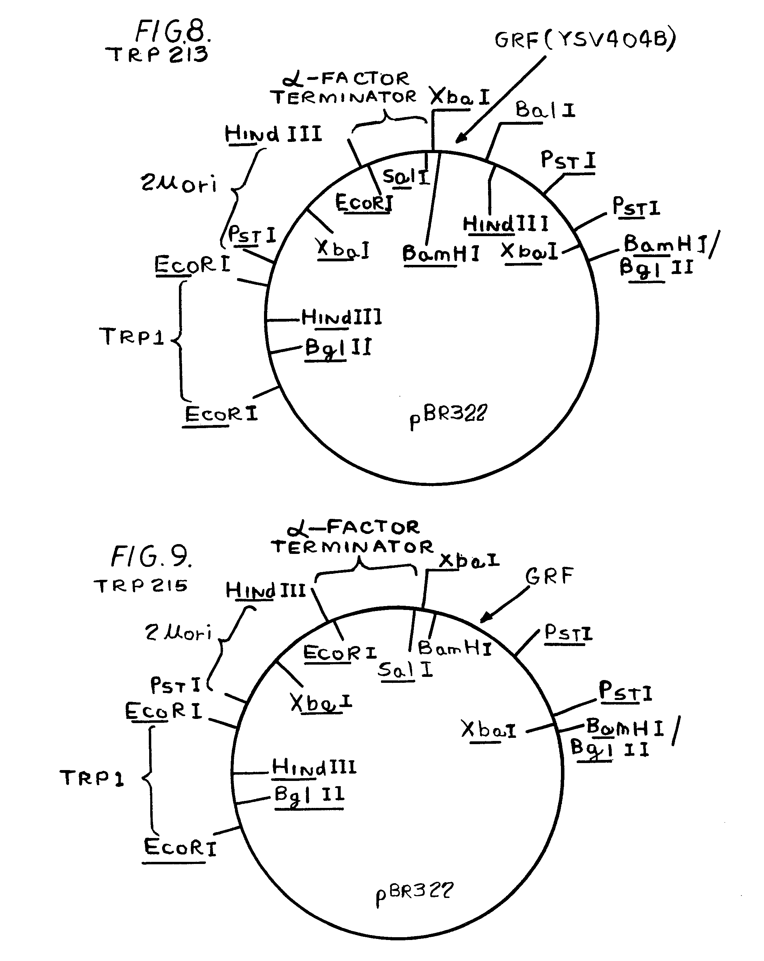

Template is prepared from these plaques as previously described, and the single-stranded DNA is attached to a nitrocellulose filter, essentially by applying the DNA to the filter through a vacuum apparatus, a slot blotter, and baking the filter. The filter was then hybridized with a probe shorter than the primer, which is capable of detecting the deletion in a hybridization experiment at the appropriate stringency. These strains harboring DNA with the desired deletion were screened via this hybridization technique, and the mutation was verified via DNA sequencing. An example of such a construct is TRP215 (FIG. 9), the construction of which is described in greater detail hereinafter.

In addition, and in accordance with an important aspect of the invention, the processing sites following the alpha-factor peptide units are used in the fusion polypeptide, as opposed to only the processing site at the carboxy-terminus of the leader peptide, to process the heterologous polypeptide from the alpha-factor peptide and promote secretion of the heterologous polypeptide. BalI sites are present in each segment of the alpha-factor structural gene encoding an alpha-factor peptide. If a plasmid containing the alpha-factor gene is cleaved with BalI and reclosed, the resulting plasmid will contain the entire promoter and a part of the structural gene, from the 5'-end thereof up to and including a part encoding a portion of the first copy of the alpha-factor peptide unit following the leader peptide. From this BalI site it is possible to create novel processing sites downstream of the alpha-factor peptide which can serve to sever peptides and proteins from alpha-factor. An example of such a construct is TRP213 (FIGURE 8), the construction of which is described in greater detail hereinafter. Of course, with BalI partial digestions and appropriate screening, more than one alpha-factor peptide could be left in the engineered gene for expression or secretion of a desired polypeptide.

Different transcription terminators were used in expression vectors. The characterization of the termination signals for the alpha-factor gene has already been described. The 3'-ward EcoRI site of the EcoRI-SalI fragment was converted to a HindIII fragment in the following fashion. A 380 bp EcoRI-HindIII fragment, representing a segment from the HindIII site in front of the fourth alpha-factor cassette to the EcoRI site following the alpha-factor transcription signals (FIGURE 1), was identified in a digest of plasmid DNA from genomic clone AMF-B. An entire EcoRI digest of AMF-B (120 ug) was treated with DNA polymerase large fragment (40 U) in the presence of 50 mM NaCl, 10mM MgCl$_2$, 10mM Tris-HCl, pH 7.4, 40uM TTP, and 40 uM dATP for 30 minutes at 20°C, to create a flush-ended fragment. HindIII linkers (6 ug) were phosphorylated as previously described for oligonucleotide labeling, except that gamma-$^{32}$P ATP was omitted and unlabeled ATP was included at a concentration of 1mM. The flush-ended EcoRI digest and phosphorylated linkers were then combined and ligated overnight at 15°C in the presence of 1200 Units of T4 DNA ligase. This ligation reaction mixture was phenol-extracted and ethanol-precipitated. The DNA was subsequently digested with 700 Units of HindIII for 5 hours. Gel electrophoresis of the digest confirmed that the HindIII digest was complete. It was digested with SalI (150 Units) according to manufacturer's specifications for two hours at 37°C. A 300 base-pair SalI-HindIII digestion fragment was identified (by reference to molecular weight standards) on an 8% polyacrylamide gel which had run for 2.5 hours at 200V. The band was isolated by electroelution. 80 ng of this fragment was ligated to 32 ng of a HindIII-SalI digest of plasmid pUC8 (purchased from Bethesda Research Laboratories, Inc., Gaithersburg, Maryland, U.S.A.). This plasmid had been treated with calf intestinal alkaline phosphatase (1 Unit) in 50mM Tris-HCl, pH 9,
1mM $MgCl_2$, 0.1mM $ZnCl_2$, 1mM spermidine for
30 minutes at 37°C. Following this dephosphorylation,
the plasmid was phenol-extracted and
ethanol-precipitated. This ligation reaction was
incubated at 14°C for 16 hours and used to transform
competent E. coli JM103 to ampicillin resistance.
Transformants containing the 300 base pair SalI-HindIII
fragment were identified. The plasmid pUC8 has a BamHI
site very the SalI site in its polylinker
segment. Con tly, the alpha-factor terminator
sequence could be cloned into a plasmid designated
TRP203 (FIGURE 3, the construction of which is to be
described in greater detail hereinafter) as a
HindIII-BamHI segment, with the BamHI site at the
5'-side of the terminator fragment.

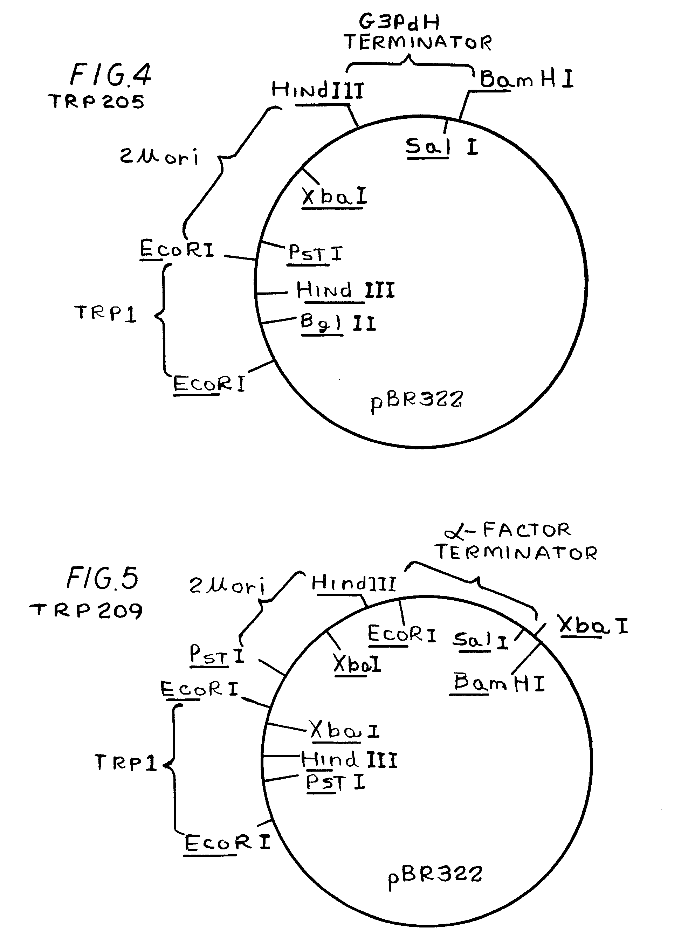

To obtain this construct, the TRP203 plasmid
was digested to completion with BamHI. This linear
molecule was partially digested with HindIII and the
longest HindIII to BamHI fragment (approximately 7500bp)
isolated from agarose gels via electroelution.
Following phenol-extraction and ethanol-precipitation,
it was treated with calf intestinal alkaline
phosphatase. The alpha-factor transcription terminator
fragment, a BamHI-HindIII fragment, was ligated to the
BamHI-HindIII-digested TRP203. This mixture was used to
transform E. coli strain MC1061 to ampicillin
resistance. These transformants were then tested for
the presence of the appropriate fragment. This vector
is called TRP209 and is mapped in FIGURE 5.

In addition to the alpha-factor terminator,
sequences from the 3'-end of the S. cerevisiae
glyceraldehyde-3-phosphate dehydrogenase gene (G3PdH)
which reside on a 200bp SalI-HindIII fragment, as
described by Musti et al., Gene 25:133-144, (1983) have
been used. A 175bp SalI-HindIII fragment was isolated,
subcloned into M13mp8, and sequenced via the Sanger dideoxy method. The inserts were then isolated from the double-stranded plasmid form of M13mp8 via restriction digestion with HindIII and BamHI, followed by electrophoresis on 10% acrylamide. The band of the appropriate size was identified by reference to molecular weight markers (a HaeIII digest of phiX174 phage DNA), after visualization by ethidium bromide staining and UV excitation. The band was excised from the gel, and the DNA was recovered by electroelution as previously described. The fragment was then cloned into the plasmid TRP203 which had been completely digested with BamHI, and then partially digested with HindIII. To accomplish this, 50 ug of TRP203 was digested to completion in BamHI, phenol-extracted and ethanol-precipitated. For partial digestion with HindIII, 16 ug was cut with 0.6 Units of HindIII for 20 minutes at 37°C.

The vector was isolated by gel electrophoresis on 0.6% agarose and a band of the appropriate size (approximately 7.5Kb) was determined by reference to molecular weight markers (a HindIII digest of bacteriophage lambda DNA). This band was visualized and excised from the gel. It was recovered via electroelution and quantitated as previously described. To create the vector called TRP205 (Mapped in FIGURE 4) wherein the HindIII-BamHI fragment of the tetracycline-resistance gene of TRP203 is replaced by the HindIII-BamHI fragment of the G3PdH transcription terminator, 5 ng of the vector was ligated to 10-25 ng of the fragment. The ligation reaction was incubated at 23°C for 3 hours. The reaction mixture was then used to transform E. coli strain MC1061 to ampicillin-resistance. Transformants were analyzed by HindIII-BamHI digestion and a clone containing the 175bp HindIII-BamHI fragment was identified.

Also used fortuitously were vector sequences from a S. cerevisiae 2u circle to terminate transcription of the alpha-factor-based constructs. In TRP203, the EcoRI-HindIII fragment contains the replication origin from the 2u circle. The sequence of 2u circle was determined by Hartley and Donelson, Nature 286:860-864 (1980). This predicts an open reading frame (subsequently discovered to encode the FLP gene) which terminates in the 2.2 Kb EcoRI fragment of the B form of 2u circle (the plasmid exists in two forms identical in sequence but differing in location of the restriction sites). Consequently, it was predicted that when genes are cloned in the BamHI site of TRP203 and are transcribed in the direction of the HindIII end of the 2u circle fragment, the transcripts terminate within 200-300 bp downstream of the HindIII site.

A DNA fragment coding for human pancreatic GRF was purchased from Creative Biomolecules as a chemically synthesized EcoRI-BamHI fragment inserted into plasmid pUC9 and designed specifically for expression in E. coli. The fragment codes for 44 amino acid GRF with an artificial initiator methionine. Following the codon for the C-terminal Leu of the 44aa hGRF peptide is a stop codon; thus the synthetic gene encodes hGRF as a free acid as opposed to the naturally occurring C-terminal amidated molecule.

The sequence of the sense strand of the fragment, with a portion of the antisense strand and certain restriction sites indicated, is as follows:

```
                 Met Tyr Ala Asp Ala Ile
    5' - AATTCATG TAC GCA GAC GCT ATC
           3'-GTA...          _____
         EcoRI                  HgaI

    Phe Thr Asn Ser Tyr Arg Lys
    TTT ACT AAC TCT TAC CGT AAA

    Val Leu Gly Gln Leu Ser Ala
    GTT CTG GGC CAG CTG TCT GCA
            _____
              PvuII
```

```
Arg Lys Leu Leu Gln Asp Ile
CGC AAG CTT CTG CAG GAT ATC
    ‾‾‾‾‾‾‾ ‾‾‾‾‾‾‾ ‾‾‾‾‾‾‾
    HindIII  PstI   EcoRV

Met Ser Arg Gln Gln Gly Glu
ATG TCT AGA CAG CAG GGC GAA
    ‾‾‾‾‾‾‾
    XbaI

Ser Asn Gln Glu Arg Gly Ala
TCT AAC CAG GAG CGT GGC GCC
                    ‾‾‾‾‾‾‾
                     NarI

Arg Ala Arg Leu Stop
CGT GCA CGC CTG TAG - 3'
                ...CCTAG-5'
    ‾‾‾‾‾‾‾        ‾‾‾‾‾
    HgiAI          BamHI
```

This synthetic GRF gene was modified for use in alpha-factor vectors. The modifications which were performed on the GRF-alpha-factor sequences were three-fold, involving both the GRF peptide and its fusion to alpha-factor.

One modification, GRFD', (Leu$^{27}$-hGRF(1-44)-OH), carried out starting with the chemically synthesized GRF gene fragment shown above, involved changing amino acid 27 from methionine to leucine. This reduces the potential risk of methionine sulfoxidation, along with the possible concomitant loss of GRF bioactivity. The mechanics of this change also result in a third base change at amino acid 22, eliminating a HindIII site inside the GRF molecule.

The GRF molecule cloned into the plasmid pBR322 was used as the starting material for this modification. 2ug of this plasmid were digested with HindIII and XbaI according to manufacturer's specifications. Two oligonucleotides which, when paired, can be inserted into the XbaI/HindIII-cut vector to create the desired mutations, were synthesized, 5'-phosphorylated by treatment with polynucleotide kinase, and annealed.  The paired oligonucleotide is

$$5'\ AGCTCCTGCAGGATATCCTGT\ 3'$$
$$3'\qquad GGACGTCCTATAGGACAGATC\ 5'.$$

5 ng of the vector were ligated with 5ng of the oligonucleotide under standard conditions.  The products of this ligation reaction were used to transform E. coli MCl061 in the usual fashion.  Transformants were screened for the presence of two PvuII sites in the molecule.  The ligation contained a vast excess of insert to vector; consequently, to eliminate the risk of multiple insertions, the plasmid (1 ug) was digested with XbaI, followed by electrophoresis on 0.8% agarose in lxTBE to assess the extent of digestion.  The vector was religated at a concentration of 20 ng/ul, a concentration favoring reclosure of the plasmid and elimination of multiple inserts.  This ligation reaction mixture was used to transform E. coli strain MCl061 to ampicillin-resistance, following standard procedures.  Ampicillin-resistant transformants were identified and plasmid DNA from them digested with EcoRI and BamHI and subjected to electrophoresis on 8% acrylamide until the Bromphenol blue marker had run off.  Plasmids containing an insert of 140bp, as opposed to 190bp, were assumed to be correct, and called GRF-D'.

A second modification, GRF-C (hGRF(1-40)-OH), involved removing 4 residues from the C-terminus to reduce GRF to 40 amino acids.  Initially the 3'-terminal 72 bp, BamHI-HindIII fragment was subcloned in pUC8 as follows:  The GRF gene, as obtained from the supplier (as an insert in plasmid pUC9), was digested with HindIII and then with BamHI.  This digest was subjected to electrophoresis on 10% acrylamide in lxTBE for 4 hours at 50 volts.  A 72 bp band was identified by reference to standards and eluted from the gel.  A BamHI digest of pUC8 was then performed, followed by a HindIII digestion.  The vector fragments were then dephosphorylated by treatment with calf intestinal alkaline phosphatase. The ligation reaction included 10ng of vector fragments and an undetermined amount of the 72 bp fragment, the reaction was performed at 16°C for 5 hours under standard conditions. The ligation mix was then used to transform E. coli MC1061 to ampicillin resistance. The presence of the insert was verified by the presence of an XbaI site in minilysates. Additional digestions with PstI, BamHI, and HindIII were performed to verify the correctness of the construct.

The C-terminus of GRF was then modified to eliminate four amino acids. The pUC8 plasmid bearing the GRF 3'-portion was linearized with BamHI and partially digested with NarI. It was unexpected that a second NarI site existed in the vector. Nevertheless, the proximity of the NarI and BamHI sites in the GRF coding segment made it possible to isolate the desired BamHI-NarI digest fragments, because it was nearly the same size as full length, linearized pUC8. The desired fragment wase isolated using a 0.7% agarose gel in 1xTBE which had run for 20 hours at 40V. The band was identified by reference to standards and electroeluted. It was subsequently dephosphorylated with calf intestinal alkaline phosphatase using standard conditions. This vector was then paired with the following oligonucleotide:

```
5' CGCTTAGTAG 3'
3'   GAATCATCCTAG 5'.
```

This molecule can hybridize to the NarI overhang and complete the 40th amino acid. It contains a tandem stop codon and an overhang to ligate to a BamHI site.

The oligonucleotides (10 ng of each) were labeled with cold ATP under standard conditions. 5 ng of the oligonucleotide pair was ligated to 5 ng of the vector under standard conditions and used to transform E. coli strain MC1061. Ampicillin-resistant transformants were analyzed for the loss of a NarI site in the plasmid. Additionally, the clone now contained a shorter (less than 65 bp) BamHI-HindIII fragment. The GRF-analog-encoding segment in this molecule is called GRF-C. This segment was subsequently subcloned into a pBR322 based GRF plasmid containing a GRF-Encoding EcoRI-BamHI insert, essentially replacing the HindIII-BamHI C-Terminal-Encoding portion of the segment encoding the 44 amino acid version (GRFD') with the analogous portion for the 40-amino acid molecule. The GRF analog encoded by GRF-C, like that encoded by GRFD', still contains the articial initiator methionine at the N-terminus.

A third modification, GRF-E-3 (alpha-factor modification), involves eliminating the artificial methionine initiation codon at the 5'-end of the GRF-analog-encoding segment and precise linkage of the 5'-end of this segment to the 3'-end of pre-pro-alpha-factor-encoding segment. 20 ug of GRF-D' was digested with HgaI and BamHI according to manufacturer's specifications, and a fragment of approximately 118 bp was isolated by electrophoresis on 10% polyacrylamide in 1xTBE. The band of interest was identified by reference to molecular weight markers. The band was electroeluted, phenol-extracted, and ethanol-precipitated. This band was then ligated with the following paired oligonucleotide, which replaces the codons for amino acids eliminated by HgaI digestion, removes the artificial ATG for N-terminal methionine, and leaves a 5'-extension capable of ligation with the HindIII end of a segment encoding a modification of the pre-pro-alpha-factor molecule which lacks 1, 2, 3 or 4 alpha-factor peptides and 3, 2, 1 or 0 processing sites, respectively:

```
5'  AGCTTACGCAGACGCTATCT 3'
3'      ATGCGTCTGCGATAGAAATGA 5'.
```

The ligation reaction was as follows: 10 ng of the fragment and 50 ng of the paired oligonucleotide (oligo:fragment molar ratio=30:1) were ligated at 23°C for 3 hours. To eliminate the possibility of multiple insertions, the ligation reaction mixture was subject to digestion with BamHI and HindIII, each for 30 minutes. The monomerized insert was then ligated to HindIII-BamHI-digested p-alpha-factor (10 ng), at 15°C overnight. This was used to transform E. coli strain MC1061. Minipreps of ampicillin-resistant transformants were digested with PstI and a clone containing the correct insert was identified, this clone is called GRF-E-3. It contains the 5'-upstream region of alpha-factor unit (from the EcoRI site to the initiation codon) and the complete sequence encoding the alpha-factor leader. The GRF sequence is attached such that the first amino acid of GRF immediately follows the last ala of the processing site at the C-terminus of the alpha-factor leader. Thus, the gene encodes the fusion polypeptide having the formula:

alpha-factor pre-segment -
        lys-arg-glu-ala-glu-ala - [Leu$^{27}$]-hGRF(1-44)-OH.
        (    processing site    )

To create a clone capable of being ligated to a sequence that encodes the alpha-factor processing site -Lys-Arg-Glu-Ala-Glu-Ala- fused directly to a [Leu$^{27}$] hGRF(1-40)-OH sequence lacking the artificial methionine, and that contains a HindIII overhang, two distinct segments were joined together. The segment for the N-terminus of the GRF analog was derived from GRF-E3 by HindIII and XbaI digestion. This 87 bp fragment was isolated by electrophoresis on 10% polyacrylamide in 1xTBE and electroeluted. The gene segment for the C-terminal portion of the GRF analog is the XbaI-BamHI fragment from GRF-C, which is a 45 bp fragment isolated by the same electrophoretic and electroelution procedures as the N-terminal-encoding fragment. These two portions were ligated together at 16°C for 16 hours and then digested with BamHI and HindIII separately. They were then ligated into p-alpha-factor which had been digested with HindIII and BamHI under the standard conditions for ligation. The ligation reaction mixture was used to transform E. coli strain MC1061 to ampicillin-resistance. Transformants were characterized by digestion of minilysates with PvuII, looking for the appearance of a second PvuII site. Such a clone was isolated and is called YSV201.

The gene coding for the 70 amino acid IGF-I peptide, with an artificial initiator methionine at the N-terminus, was obtained from others. The gene is carried on an EcoRI-HindIII fragment. This fragment was isolated on a preparative 8% acrylamide gel, visualized and electroeluted. The 220-bp band was subsequently digested with AvaII which cuts at the codon for IGF-I amino acid #2. The extent of digestion was determined by comparing the digested with undigested material on a 10% acrylamide gel.

The following oligonucleotide pair was synthesized for use in modifying the IGF-I gene for insertion into an alpha-factor-encoding vector:

```
5' AGCTAAGAGAG 3'
3'     TTCTCTCCAG 5'
```

When this paired oligonucleotide was ligated to the AvaII-HindIII fragment, a molecule was created that had a HindIII overhang on both ends, but a HindIII site only at the C-terminal encoding end. For this reason, only the antisense strand of this oligonucleotide pair was kinased using standard kinasing procedures. This allows the oligonucleotide pair to ligate to the fragment, but once ligated, the fragment cannot ligate to itself at the N-terminal encoding end. This is an important consideration because, if this precaution were not taken, the fragment would then become resistant to HindIII digestion.

For this oligonucleotide-fragment ligation, 60 ng of fragment was ligated to 30 ng of paired oligonucleotide at 16°C for 18 hours using standard reaction conditions. The resulting mixture was then digested with HindIII to resolve any dimers which may have been formed due to ligation at the HindIII site at the C-terminal-encoding portion of this molecule. The resulting mixture was then ligated to 20 ng of a HindIII digest of p-alpha-factor for 1 hour at 23°C. The ligation reaction was used to transform E. coli strain MC1061 to ampicillin-resistance, and the transformants were screened for the presence of plasmid with a BstEII site. Only three out of twelve transformants had plasmid with such a site. These were subsequently digested with BamHI to determine which had the correct orientation. Only one of the three insert-containing clones had the correct orientation. This plasmid is called YSV301.

To make YSV301 compatible with the alpha-factor expression vector, TRP209, the plasmid YSV301 was digested with HindIII and fragments rendered flush-ended by treatment with DNA polymerase large fragment, as previously described. Following phenol-extraction and ethanol-precipitation, the plasmid was ligated to phosphorylated BamHI linkers using standard conditions at 16°C for 15 hours. The sample was then diluted and digested extensively with BamHI (less than 200 Units for 6 hours). The plasmid (2.5 ng) was then religated using conditions favoring plasmid reclosure and used to transform E. coli strain MC1061 to ampicillin-resistance. Transformants were screened for the presence of a BamHI site in minilysates via BamHI digestion. One such clone was isolated and called YSV302.

The digestion of YSV302 with BamHI and BglII yields a 1300 bp fragment. This fragment was isolated from a 6% acrylamide gel by electroelution, and phenol-extracted and ethanol-precipitated. Approximately 20 ng of this fragment was ligated to 6 ng of a dephosphorylated BamHI digest of TRP209, under standard conditions and used to transform E. coli strain MC1061 to ampicillin-resistance. Transformants were analyzed by XbaI digestion of minilysates. Those with the correct orientation, i.e., the 3'-end of the IGF-I gene next to the transcription terminator, were chosen by identification of diagnostic restriction fragments relative to standards of known molecular weight. This clone is known as YSV303 and is mapped in FIGURE 2.

In the following description of vector construction, all alpha-factor vectors are derivatives of a set of 2u circle-based vectors, containing the wild type Saccharomyces cerevisiae TRP1 gene. All components are contained in a pBR322-based plasmid for selection and replication in E. coli.

The first vector in this series, TRP201, contains the 2.2kb EcoRI fragment from the "B" form of the 2u circle which has the signals necessary for replication in Saccharomyces. The insert was obtained by digesting YEp24 (available from ATCC, accession number 37051) with EcoRI. Following electrophoresis, a 2.2Kb fragment was identified by reference to standards, electroeluted and precipitated. This fragment was ligated to a dephosphorylated EcoRI digest of pBR322. The ligation reaction mixture was used to transform E. coli MC1061 to ampicillin resistance. Ampicillin resistant colonies were characterized by EcoRI digestion of minilysates. Two orientations are possible in this construction. It was chosen to use the one in which the HindIII site of 2u circle is closest to the HindIII site in the tetracycline-resistance gene on pBR322. This clone is designated TRP201.

One EcoRI site was deleted to form a plasmid vector designated TRP202. The minilysates containing plasmid with the correct orientation (TRP201) were pooled and plasmid digested with HindIII to completion. They were then diluted to 10 ng/ul, and 10 ng were then religated in a 50 ul reaction in the usual manner. The ligation reaction mixture was used to transform E. coli strain MC1061 to ampicillin-resistance. Transformants were analyzed by EcoRI digestion of minilysates, as the reclosure of TRP201 after digestion with HindIII would have eliminated one of the two EcoRI sites.

TRP203 (FIGURE 3) was created by digesting TRP202 with EcoRI and treating it with calf intestinal alkaline phosphatase to dephosphorylate the 5'-ends of the linearized vector. A 1.45 Kb EcoRI fragment containing the TRP1 gene (and the associated ARS1 sequence) was obtained via EcoRI digestion of plasmid YRp7 (available from ATCC, accession number 37060), followed by electrophoresis on 0.8% agarose. The 1.45Kb band was identified by reference to standards. It was isolated by electroelution and precipitated. 10 ng of the vector (TRP202) was ligated to 50 ng of the 1.45 kb insert at room temperature for 1 hour.

This ligation reaction mixture was used to transform E. coli strain MC1061 to ampicillin-resistance. Transformants were analyzed for the presence of the insert and its orientation relative to the 2u circle fragment. Those in which the TRP1 sequences are closest to the 2u fragment are designated TRP203 (FIGURE 3) the other orientation is TRP204.

The construction of TRP205 (FIGURE 4) and TRP 209 (FIGURE 5) are described hereinabove.

GRFE-3 was inserted into TRP205 to form vector TRP206, which is mapped in FIGURE 6. To make this construct, TRP205 was digested with BamHI and dephosphorylated. 10 ng of this vector were ligated to a 1200 bp BamHI-BglII fragment isolated from GRFE-3 by digestion, electrophoresis and electroelution. The ligation reaction contained 25-75 ng of this fragment and was ligated at 23°C for 2 hours. This ligation reaction mixture was used to transform E. coli strain MC1061 to ampicillin-resistance. Transformants were characterized by PvuII digestion of minilysates. Those bearing the correct orientation were designated TRP206. The TRP206 vector is propagated in both E. coli and yeast, and expresses a 44 amino-acid GRF analog peptide with leucine at amino acid 27 in yeast cells.

GRFE-3 was inserted into TRP209 to form a vector, TRP210, which is mapped in FIGURE 7. TRP209 was digested with BamHI and treated with calf intestine alkaline phosphatase to prevent vector reclosure. The alpha-factor-GRF fragment isolated from GRFE-3 was ligated into TRP209. The ligation reaction was used to transform E. coli strain MC1061 to ampicillin-resistance. These transformants were screened to determine the correct orientation, i.e., the GRF gene inserted between the 5'- and 3'-regions of alpha-factor.

Pre-pro-alpha-factor has two segments in the processing site at the carboxy-terminus of the leader at which the alpha-factor peptide units are liberated from the leader peptide. The first of these segments is a pair of basic amino acids (lys-arg). The second has the sequence glu-ala-glu-ala. It is known that cleavage at the basic amino acids is more efficient than at the sequence glu-ala-glu-ala. To improve processing around the N-terminus of the GRF molecule, a sequence encoding lys-arg was placed next to the GRF-encoding sequences for two constructs, TRP211 and TRP213. TRP211 has an identical restriction map to TRP210, shown in FIGURE 7.

The processing site, coded by TRP211 and fused to the N-terminus of the heterologous protein coded by the plasmid and to be secreted, will read lys-arg-glu-ala-glu-ala-lys-arg. The GRF moiety encoded by TRP211 is a 44-amino acid polypeptide with a free acid carboxy-terminus and with methionine at position 27. The starting material for construction of TRP211 is the synthetic GRF gene from Creative Biomolecules, cloned into pBR322 as a BamHI-EcoRI fragment. The plasmid with this gene was digested with BamHI and HgaI as previously described. To this fragment was added the segment:

                5'   AGCTAAAAGATACGCAGACGCTATCT        3'
                3'            TTTTCTATGCGTCTGCGATAGAAATGA 5'

in the following fashion:  the antisense strand (180 ng) of the segment was phosphorylated as previously described using T4 polynucleotide kinase and gamma-$^{32}$P ATP and chased with cold 1mM ATP.  The product of this reaction was purified by Sephadex G-50 chromatography, and the enzyme was heat inactivated at 65°C.  150 ng of sense strand of the segment was next added to this mixture and the mixture allowed to slowly cool to room temperature.  180 ng of the product of this reaction was ligated to 90 ng of the isolated HgaI-BamHI fragment at 16°C for 16 hours.  In this scheme, the end with the HindIII overhang is incapable of self-ligation (such a dimer would be incapable of digestion with HindIII), hence only dimers of the fragment ligated at the BamHI site can be generated during ligation.  The ligation mix was then cut with 5 Units of BamHI for 20 minutes at 37°C.  This mixture was phenol-extracted, 5 ug of carrier tRNA was added and the nucleic acid was ethanol-precipitated.  This fragment is now capable of ligation to a vector which has been digested with HindIII and BamHI.  In this case, the vector is again p-alpha-factor.  The ligation reaction contained less than 5 ng of vector and the 90 ng of insert prepared by the ligation, just described, of the double-stranded oligonucleotide to the HgaI-BamHI fragment  of the synthetic GRF gene.  The products of this ligation reaction were used to transform E. coli strain MC1061 to ampicillin-resistance.  Transformants were analyzed by PvuII digestion of DNA minilysates, followed by electrophoresis on 0.8% agarose gel and comparison of fragment sizes to fragments of known molecular weight. A transformant bearing the correct spectrum was isolated and called GRFI.

0206783

The GRFI plasmid was digested with BamHI and BglII and a 1200 bp fragment was identified and isolated by electroelution. This fragment was then cloned into the BamHI site of TRP209. Plasmids with the fragment in the correct orientation were identified and called TRP211 (FIGURE 7). This plasmid is identical to TRP210 except that it has an additional pair of codons for basic amino acids (lys-arg) between the coding sequences for glu-ala-glu-ala and the GRF. The encoded fusion polypeptide has the formula:

leader pre-segment -
    lys-arg-glu-ala-glu-ala-lys-arg-[Met$^{27}$]-hGRF(1-44)-OH.
    (        processing site        )

The processing signals of alpha-factor-driven heterologous genes were also altered by placing the sequence lys-arg behind the first alpha-factor peptide to which the GRF peptide is attached. This construct was accomplished in the following fashion, with the series of constructs: YSV401; YSV402; YSV403, YSV404B; TRP213, a map of which is shown in FIGURE 8.

The 1.7 kb EcoRI fragment encoding the complete alpha-factor gene was isolated from genomic clone AMF-B. This was accomplished by digesting the genomic clone with EcoRI and subjecting the digest to electrophoresis in 0.7% TBE agarose. The 1.7 Kb fragment was identified by reference to DNA molecular weight standards. The band was excised, and the DNA was electroeluted and precipitated. The fragment (50 ng) was then ligated, at room temperature for 2 hours, into 10 ng of a dephosphorylated EcoRI digest of pBR322, and the ligation reaction mixture was used to transform E. coli strain MC1061 to ampicillin-resistance. Plasmid from ampicillin-resistant transformants was digested with EcoRI and subsequently with SalI to determine the insert orientation. The preferred orientation is that in which the SalI site at the 3'-end of the alpha-factor gene is closer to the SalI site in pBR322. This plasmid is designated YSV401.

Plasmid YSV401 is then digested with BalI and religated. 2 ug of YSV401 was digested with BalI according to manufacturer's specifications. The linear band was isolated following electrophoresis on 0.7% agarose. This band was then religated to itself under standard conditions favoring reclosure. Such a plasmid, having deletion of sequences from the BalI site in the coding sequence for the first alpha-factor peptide and all sequence downstream thereof, including the entire tetracycline-resistance gene of pBR322, is called YSV402.

Plasmid YSV402 was linearized with PvuII and was ligated to phosphorylated BamHI linkers under standard conditions. The products of the ligation reaction were then extensively digested with BamHI to remove polymers of BamHI linkers attached at the PvuII site and to generate the BamHI sticky ends. The vector was then religated and subsequently digested with PvuII to eliminate any molecules which had not taken up the BamHI linkers. The products of the religation and digestion were used to transform E. coli strain MC1061 to ampicillin-resistance. Transformants were tested for the presence of plasmid with a BamHI site, the absence of a PvuII site and the correct linear fragment size. This plasmid is called YSV403.

The precursor of TRP213 is called YSV404-B and is made in the following fashion. The construct YSV403 was digested with BamHI and BalI separately. The linear band was isolated from a 0.7% agarose gel by electroelution. The plasmid YSV201 was constructed as previously described. This plasmid was digested with BamHI and HgaI. The 110 bp fragment was isolated via electrophoresis on 8% acrylamide followed by electroelution and precipitation.

The following oligonucleotide was added to the 110 bp fragment by phosphorylating both strands of the oligonucleotide and ligating a 20-fold molar excess of the oligonucleotide to 15 ng of the fragment for 5 hours at 16°C:

```
alpha-factor peptide lys-arg  GRF---
    5' CCAACCAATGTAC AAA AGA  TACGCAGACGCTATCT 3'
    3' GGTTGGTTACATG TTT TCT  ATGCGTCTGCGATAGAAATGA 5'.
```

The products of this ligation reaction were then added to the 10 ng of BalI-BamHI-digested YSV403 and the mixture was used to transform E. coli strain MC1061 to ampicillin resistance. Transformants were picked onto L-B ampicillin agar plates and onto nitrocellulose filters on top of L-B ampicillin plates. These filters were then prepared for hybridization as previously described. They were hybridized with the sense strand oligonucleotide,

5'-CCAACCAATGTACAAAAGATACGCAGACGCTATCT-3', which had been radiolabeled with gamma-$^{32}$P ATP as previously described. The hybridization was performed in 5xSSPE, 0.5% SDS and 1 mg/ml denatured salmon sperm DNA, with $3 \times 10^5$ cpm/ml in a volume of 10ml at 65°C for 16 hours. The filters were then removed from the bag and washed two times in 2xSSC, 0.5% SDS at room temperature for 5 and 20 minutes respectively. The filters were then washed at 65°C for 15 minutes, air dried and exposed to Kodak XAR-5 film for 3 hours, with an intensifying screen at -70°C.

Four positive clones were identified and analyzed in minilysates by digestion with BamHI and BglII. Three of the clones gave apparently identical spectra consistent with the correct construction. One of these was called YSV404-A. This clone was digested with PstI and yielded a fragment of around 500bp. This is consistent with the presence of three alpha-factor peptides in the clone. This undoubtedly arose by partial digestion with BalI during the construction of YSV402. The clone YSV404-A was digested with HindIII to eliminate two of the alpha-factor peptides. 250pg was then ligated in a volume of 10 ul for 3h at 23°C and used to transform E. coli MC1061 to ampicillin-resistance. Two clones were analyzed for the correct size band in a PstI digest. One of these clones, of correct size, is designated YSV404-B. The precision of the fusion of GRF coding sequence with alpha-factor coding sequence in YSV404-B was confirmed by Sanger dideoxy sequencing.

Plasmid YSV404-B was digested with BamHI and BglII, and the 1250 bp fragment was isolated by agarose gel electrophoresis followed by electroelution. Approximately 50 ng of the fragment was ligated to 5 ng of a dephosphorylated BamHI digest of TRP209. The ligation mixture was incubated at 23°C for 2 hours and then used to transform strain MC1061 to ampicillin resistance. The transformants were screened by the same colony hybridization technique used to identify YSV404-2. Two clones were identified and called TRP213 (FIGURE 8). They include the 5'-end of alpha-factor gene, including the sequence coding for the complete pre-pro-alpha-factor leader segment and the first alpha-factor peptide. In the fusion protein encoded by this sequence, the first alpha-factor peptide is joined at its carboxy-terminus to the dipeptide lys-arg which, in turn, is joined at the arg carboxy group to the peptide segment glu-ala-glu-ala, which in turn is joined at the carboxy-terminal ala to the N-terminal tyr of [Leu-27]GRF(1-40)-OH.

In order to effect a deletion of the sequences encoding the glu-ala-glu-ala segment of the processing site between the alpha-factor peptide and the GRF analog in the alpha-factor-GRF fusion polypeptide encoded by TRP213, a 1.2kb EcoRI-BamHI fragment from YSV201 was subcloned into Ml3mpl8. The M13 mutagenesis protocol was employed as described below to remove the 12 bases coding for glu-ala-glu-ala. The primer was homologous to the antisense strand of the alpha-factor GRF fusion junction:

```
3' CTT CTT CCC CAT AGA AAC CTA TTT TCT-
   GAA GAA GGG GTA TCT TTG CAT AAA AGA-
   glu glu gly val ser leu asp lys arg-

                             ATG CGT CTG 5'
           AAA AGA GAG GCT GAA GCT TAC GCA GAC
           glu ala glu ala tyr ala asp
           deleted deleted                        .
```

This mutagenesis protocol eliminated the sequences coding for the glu-ala-glu-ala residues. Mutants with the desired deletion were selected by hybridization with the $^{32}$P-labeled oligonucleotide:

```
           5' GTCTGCGTATCTTTTATCCAAAGA 3'    .
```

Following the mutagenesis, double-stranded, replicative-form DNA was prepared and the DNA was digested with BamHI and BglII. The fragment was isolated and subcloned into the BamHI site of TRP209. Those clones containing the insert in the correct orientation are called TRP215, which is mapped in FIGURE 9.

To remove sequences which might interfere with the correct processing of IGF-I from alpha-factor pre-segment, a version of YSV303 is constructed with a modified processing site. YSV303 (FIGURE 2) contains a segment encoding the polypeptide sequence:

```
           alpha-factor pre-segment ...
               lys-arg-glu-ala-glu-ala-lys-arg-IGF-I
               (processing site             )
```

One copy of lys-arg and the sequence glu-ala-glu-ala have been eliminated.

To accomplish this task, the EcoRI-BamHI fragment from YSV302 was subcloned into M13mp18. YSV302 was digested with EcoRI and BamHI and subjected to electrophoresis through 0.8% agarose in 1xTBE. A

1300 bp band was identified and electroeluted. A 20 ng aliquot was ligated with 10 ng of a BamHI-EcoRI digest of M13mp18 which had been dephosphorylated with calf intestinal alkaline phosphatase. The ligation reaction was allowed to proceed at room temperature for several hours and the resulting products cloned into E. coli JM103. Plaques were characterized by EcoRI-BamHI digestion of the double-stranded DNA of replicative form of the M13 phage genome. Phage bearing the correct insert were grown up using standard techniques and single-stranded template was prepared. The single-stranded template bearing the sense strand was annealed with a primer bearing the following sequence in 1 x HindIII buffer (20 mM Tris-pH 7.5 10mM MgCl$_2$, 50mM NaCl, 1mM dithiothreitol) by heating to 65°C and slow cooling to room temperature.

```
template   5' GTATCTTTGGATAAAAGAGAGGCTGAAGCT-
primer     3'        AGAAACCTATTTTCT              -

AAAAGAGGTCCCGAAACTCTG 3'
        CCAGGGCTTTGAGAC 5'   .
```

The annealing reaction was carried out in 20mM Tris-HCl, 10mM MgCl$_2$, 10mM dithiothreitol (DTT), 25mM NaCl, 500uM ATP, 10u Ci $^{32}$PdATP, 500uM of dCTP, dGTP, TTP, 4uM dATP and 3 Units of T4 ligase and 2.5 Units of DNA polymerase (large fragment). The reaction was incubated at room temperature for 5 minutes, and 1 ul of 10mM dATP was added. The reaction was then incubated at 15°C for 12-20 hours.

The reaction mixture was then precipitated with a 1:1 mixture 1.6M NaCl/13% PEG 6000 to precipitate closed circular DNA, and resuspended in 200ul 10mM Tris pH 8, 1mM EDTA.

An alkaline sucrose gradient (5-20% sucrose, 1M NaCl, 0.2M NaOH, 2mM EDTA) was prepared and run in a SW50.1 rotor. The samples were adjusted to 0.2 N NaOH and the gradients were run at 37,000 RPM for 2 hours at 4°C. The gradients were pumped out and the fractions counted. Fractions in the bottom half of the gradient were pooled and neutralized with 1M Tris-Citrate pH 5. They were subsequently dialyzed against TE. These samples were then used to transform E. coli JM103.

Transformants were grown and templates were isolated using a miniprep procedure and dissolved in 50 ul TE. 5 ul were spotted onto a nitrocellulose filter in a slot blot apparatus. The filter was baked and then hybridized with a radiolabeled oligonucleotide probe using standard procedures. In this case, the probe was 3' CTATTTTCTCCAGGGCTT 5'  .

Hybridization was carried out at 23°C in 6 x SSPE, 10 x Denhardt's (2% bovine serum albumin, 0.2% Ficoll, 0.2% polyvinylpyrrolidone), 0.2%SDS. There was a 1 hour prehybridization at 67°C prior to hybridization. About 500,000 cpm/ml was used in the hybridization, which was carried out for 1 hour at 23°C. The filter was then washed in 6 x SSC at 23°C and exposed to film for 1 hour. It was washed at successively higher temperatures until hybridization was observed in a plus-minus fashion relative to a control template (i.e. the starting material). These positive samples were then grown in a large scale culture, and the double-stranded DNA replicative intermediates were prepared in the usual fashion. This DNA was then digested with BamHI and BglII, and the fragment containing the novel alpha-factor-IGF-I sequences were isolated in the usual fashion and ligated to BamHI-digested TRP209. This ligation reaction mixture was used to transform E. coli strain MC1061 to ampicillin-resistance and plasmid in transformants was analyzed by XbaI digestion of minilysates. Those plasmids bearing the correct orientation are designated YSV304.

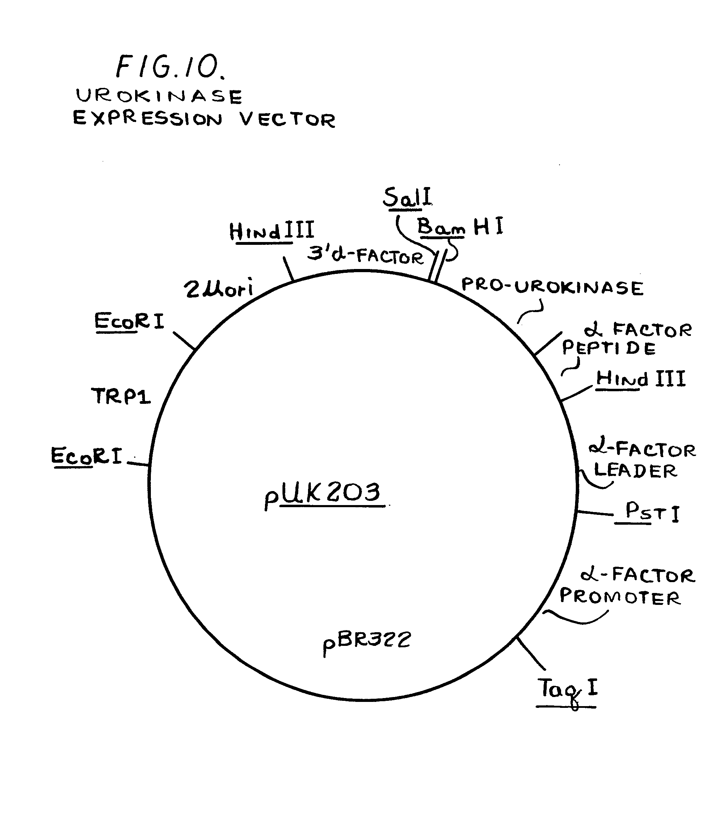

Illustrated in FIGURE 10 is a plasmid vector, designated pUK203, for expression in S. cerevisiae of a fusion protein including human prourokinase at its carboxy-terminus. This vector includes a recombinant DNA sequence that encodes,from a gene transcribed from the alpha-factor gene promoter and including the alpha-factor gene terminator, the fusion polypeptide:

alpha-factor leader pre-segment-
    lys-arg-glu-ala-glu-ala
    (    processing site    )

alpha-factor peptide
    lys-arg-prourokinase.
    (processing).
        site

     pUK203 was made as follows starting with a TagI-HindII fragment encoding the portion of human prourokinase, beginning with amino-acid 9 (see, e.g., FIGURES 4A and 9 of published Great Britain Patent Application No. 2,121,050), and the pre-pro-alpha-factor gene of clone AMF-B.

     Employing standard site-directed mutagenesis techniques, the -GGC- codon for glycine in the coding segment for the fourth alpha-factor peptide of the alpha-factor gene was converted to a different glycine codon, -GGG-. This created a SmaI site in this coding segment.

     An EcoRI-SmaI fragment including the alpha-factor promoter and coding sequence to the newly created SmaI-site in the coding segment for the fourth alpha-factor peptide was then subcloned into pUC18, that had been cut with EcoRi and SmaI. The resulting plasmid was then digested with HindIII and then reclosed and transformants with reclosed plasmid were screened for plasmid in which all but the first (i.e., the 5'-most) of the four alpha-factor-peptide coding segments had been deleted.

     The TaqI-HindIII fragment with part of the prourokinase gene was cloned into pUC18 that had been cleaved with AccI and HindIII.

     The pUC18 vector with the prourokinase-encoding insert was cleaved with TaqI and HindIII and the prourokinase-encoding fragment isolated and then ligated to the adapter

5'-CCGGGCAACCAATGTACAAGAGAAGCAATGAACTTCATCAAGTCCCAT-3'
  3'-CGTTGGTTACATGTTCTCTTCGTTACTTGAAGTAGTTCAGGGTAGC-5'

This adapter includes: a TaqI sticky end at its 3'-end; a series of 8 codons, that code for the first eight amino acids of prourokinase, followed by a T at the 3'-end that corresponds to the T of the codon -TCG- for the 9th amino acid; the segment AAGAGA, which codes for a lys-arg processing site fused to the N-terminal serine of the prourokinase; an XmaI sticky end at the 5'-end; and, beginning with the XmaI sticky end, the sequence

     5'-CCGGGCAACCAATGTAC-3', which includes the last two bases for the codon for proline at position-8 of alpha-factor peptide and continues with the codons for amino acids 9-13 of the alpha-factor peptide. Following the ligation, and isolation of the desired fragment the resulting fragment is cloned in XmaI-HindIII-cleaved pUC18. The fragment was then isolated from the pUC18 by cutting with XmaI and HindIII and was ligated to the pUC18 plasmid that included the alpha-factor insert between the EcoRI and SmaI sites and that had been linearized with XmaI. The resulting plasmid included, on an EcoRI-HindIII fragment, in order from 5' to 3', the alpha-factor promoter and structural gene through the segment encoding the first alpha factor peptide, codons for the processing site lys-arg, and the segment encoding prourokinase.

The HindIII-site at the 3'-end of the fragment was converted to a SalI site, following standard procedures, by cutting with HindIII, isolating the EcoRI-HindIII fragment with the alpha-factor-prourokinase fusion gene, filing in and ligating to a SalI adapter,

     5'-GGTCGACC-3'
     3'-CCAGCTGG-5'

The BglII site near the 5'-end of the fragment and the 5'-end of the alpha-factor promoter (see FIGURE 1) was converted to a XhoI site by cutting the fragment with BglII, isolating fragment of appropriate size, filling in and ligating to a XhoI adapter,

5'-CCTCGAGG-3'

3'-GGAGCTCC-5'.

Finally, pUK203 is made by cloning the XhoI-SalI fragment with the alpha-factor-prourokinase fused gene into the SalI site of plasmid TRP209. The ligation of a 5'-XhoI overhanging end with the 3'-SalI overhanging end leaves a TaqI site near the 5'-end of the alpha-factor promoter in pUK203 (See FIGURE 10).

For production of biologically active prourokinase, the pUK203 plasmid is transformed into the GG100-14D strain of S. cerevisiae yeast, in the manner described above with respect to GRF-encoding vectors, and the transformants, selected on the basis of the TRP1 gene, are cultured.

Most examples in this application deal with the Saccharomyces cerevisiae yeast strain GG100-14D. The strain was constructed by crossing strain D13-1A (Mat a his3-532, trpl, gal2, suc2) with DB4 (Mat alpha, pho5, ura3-52). One haploid strain of this cross is GG100-14D with the phenotype (Mat alpha, trpl, his3-532, ura 3-52, pho5).

Yeast transformation, with GG100-14D or other strains referred to in the present application, is accomplished as described in the following protocol.

Yeast transformation with hybrid plasmid DNA.

NOTE:   All procedures at room temperature except as noted.

day 0:   1.   Inoculate a 5 ml culture tube with a loopful of cells. Do this in early morning.

2. When cells have reached late log phase (more than $10^7$ cells/ml) inoculate 500 ml YEPD (1% yeast extract, 2% peptone, 2% dextrose) with $10^7$-$10^9$ cells. This should be done in the evening.

day 1: 1. Cells should be harvested at no more than $2 \times 10^7$/ml ($10^7$/ml is better), as transformability decreases with increased cell density in culture. Harvest in sterile 500 ml bottles, 250 ml per bottle, 4000 rpm, 5 min.

2. Resuspend pellet in 25 ml 1M Sorbitol - Remove 0.1 ml for live cell count (see below).

3. Spin at 5000 rpm, 5 min.

4. Resuspend in 25 ml 1M Sorbitol - Remove 0.1 ml for live cell count (see below).

5. Add 1 ml glusulase for spheroplasting, shake at 30°C for 1-2 hours. (Glusulase is purchased from Endo Laboratories, Wilmington, Delaware, U.S.A.)

6. Assay spheroplasting by diluting 10X cell cultures into drop of 5% SDS on microscope slide and observe "ghosts".

7. After spheroplasting, do live cell count again.

8. Spin out spheroplasts - 2500 rpm, 3 min.

9. Wash once with 1M Sorbitol.

10. Wash again in STC (1M Sorbitol, 10 mM Tris, 10 mM $CaCl_2$, pH 7.5).

11. Resuspend in 1.0 ml STC.

12. Add 0.5 ml spheroplasted cell suspended to each of two large disposable test tubes.

13. To each tube, add 20 ul of DNA solution (DNA concentration between about 250 pg/ul to about 250 ng/ul), mix, let sit 10 min.

14. Add 4.5 ml 40% PEG 10, mM $CaCl_2$ to each tube, mix by inverting tubes. Cells should become clumpy. Wait 10 min.

15. Spin in table top centrifuge at low speed ( 2500 rpm).

16. Resuspend in 5 ml STC. Add to a 100 ml bottle of regeneration agar (see below) at 50°C.

17. Mix by inverting and pour into three empty petri plates.

18. Incubate three days at 30°C.

Regeneration Agar (per liter):

    182 g Sorbitol
    20 g agar
    6.7 g Yeast Nitrogen Base without amino acids.
    20 g glucose
    1 ml liquid YEPD (optional)
    amino acid additions as necessary (depending on
        requirements of yeast strain).

Live cell count:

1. 0.1 ml cell suspension placed into 10 ml sterile $H_2O$.

2. Dilute 1000-fold in $H_2O$ (dilute only 10-fold after spheroplasting).

3. Spread 0.1 ml on YEPD plate.

4. Count

Following transformation, the transformants, which have been selected for reversion to tryptophan prototrophy, are stored on synthetic plates lacking tryptophan. The plates are composed of the following components: 0.67% yeast nitrogen base without amino acids, 2% glucose, and 2% agar. Amino acids and nitrogenous bases are added as needed in the following concentrations, in mg/l indicated in parentheses: adenine (20), uracil (20), L-tryptophan (20), L-histidine (20), L-Methionine (20), L-tyrosine (30), L-leucine (30), L-isoleucine (30), L-lysine (30), L-phenylalanine (50), L-glutamic acid (100), L-aspartic acid (100), L-valine (150), L-threonine (200), L-serine (375). Of course, as understood by the skilled, for transformants selected on the basis of prototrophy for any of these bases or acids, that base or acid will not be included or will be added at a concentration far below that required for growth of non-transformants.

When the transformants have grown on the master plate, they are stored at 4°C in petri dishes sealed with parafilm.

The efficiency of secretion is determined by taking the clarified media from a culture of a transformed yeast (i.e., the supernatant left after pelleting an aliquot of culture by centrifugation) and assaying the clarified media for the desired product. The cells are then harvested and split into three fractions. The first is the spheroplast fraction wherein a 25 ml aliquot of culture is pelleted and the cells are treated with 20 ul of glusulase in the presence of 2 ml of 1M sorbitol for 30 min at 30°C. The supernatant from the centrifugation of this extract is called the spheroplast fraction. The pellet is then taken up in 2 ml of 10 mM Tris-HCl, pH 7.5/0.2% Triton to lyse the cells and release their membranous components. This is called the membrane fraction.

A cytoplasmic fraction is made by pelleting 25 ml of culture and redissolving the pellet in 3 ml PMSF buffer (10mM Tris-HCl, pH 7.5, 1mM EDTA, 1mM PMSF (phenylmethylsulfonyl flouride)). 2 grams of glass beads are added and a lysate is made by vortexing for 2 minutes at 15 second intervals, with 15 seconds on ice between the intervals. The supernatant is then removed and the pellet is washed with 1 ml of PMSF buffer, with the wash added to the previously removed supernatant. The fractions are then assayed for immunoreactivity, as in a radioimmunoassay (RIA) with an anti-GRF antibody and radiolabeled, authentic GRF. Because the membrane fraction also contains the cytoplasmic fraction, the cytoplasmic fraction is subtracted from the membrane fraction, yielding a value for membrane-associated fraction only. The percent secretion is simply a ratio of the amount of activity found in the media, divided by the total activity. Total activity is the sum of activities found in media, spheroplast fraction, membrane-associated fraction and cytoplasmic fraction.

Transformants are grown under continuous fermentation conditions; cells are collected every day, and the cells and debris are removed by filtration. The resulting clarified media containing recombinant GRF is acidified and concentrated by batch adsorption to a C18 resin. The concentrated material is desalted, followed by fractionation on HPLC. This procedure yields more than 50% RIA-active material.

RIA analysis of the media from pTRP213-transformed GG100-14D measures 20-30 mg secreted GRF/liter.

Amino acid compositional analysis indicates that the GRF peptide, [Leu-27]-hGRF(1-40)-OH, secreted by TRP213-transformed GG100-14D, and purified with a high pressure reverse phase column, is closely similar to a synthetic polypeptide standard, hGRF(1-40)-OH (with Met at position 27), with less than a 10% average variance between mole % values for the individual amino acids. Moreover, the absence of histidine and very low levels of proline and methionine, amino acids which are not present in the peptide sequence, strongly confirm the identity of the recombinant product as that expected from the gene sequence. Additionally, the levels of contaminating proline and methionine calculate to a lower limit of 80% purity for the peptide from yeast.

A rat pituitary cell bioassay for GRF was conducted using the purified GRF analog secreted from pTRP213-transformed S. cerevisiae cells (GG100-14D). The ability of the GRF analog to cause the release of growth hormone from the pituitary cells was measured and compared to the ability of a synthetic hGRF(1-40)-OH standard to do the same. Such an analysis allowed the biological activity of yeast-produced GRF analog to be evaluated.

Typically, pituitaries of 20 rats were removed and treated with collagenase to obtain a suspension of approximately $8 \times 10^7$ cells which were then plated in the appropriate medium. The cells, attached to tissue culture dishes, were treated with yeast-derived peptide or synthetic standard within three to five days after plating. Dexamethasone was added to enhance the effect of GRF on GH secretion; cells treated in this manner secrete 8-9 times more GH than do control cells treated with GRF but not dexamethasore. Growth hormone secretion levels were measured using an anti-rat GH RIA which had been standardized with respect to the synthetic standard.

The percent of maximum rat growth hormone secretion upon treatment of the plated pituitary cells with purified GRF analog secreted from yeast and treatment with synthetic hGRF(1-40)-OH was determined. The $ED_{50}$ value for secretion with both treatments was about 32 pM.

The equivalency of the $ED_{50}$ values for the yeast-derived GRF analog and synthetic GRF standard demonstrate the full biological activity of the yeast-derived GRF analog, secreted from pTRP213-transformed Saccharomyces cells.

The media from GG100-14D yeast transformed with vector YSV303 and grown in a continuous fermentation culture was analyzed for the presence of secreted IGF-I. ELISA (enzyme-linked immunosorbent assay) analysis demonstrated 70-80 mg IGF-I/liter media.

In addition to the secretion results observed with GG100-14D transformants, alpha-factor leader peptide-facilitated secretion of product polypeptide is found to occur in certain mutants of a-type strains of S. cerevisiae yeast, a surprising and totally unexpected result. The alpha-factor mating pheromone in nature is found to occur exclusively in alpha-strains of
S. cerevisiae yeast and not at all in a-strain of
S. cerevisiae yeast. It is believed that the a-mating
type of S. cerevisiae yeast carries the gene for the
alpha-factor pre-pro-peptide but that various mechanisms
in a-strains prevent this alpha-factor gene from being
expressed. Whatever these mechanisms are, they appear
to be very effective, as secretion of alpha-factor from
an a-strain has not been previously reported.
Accordingly, the finding that a recombinant vector which
includes the DNA sequence encoding the alpha-factor
sequences linked to a gene of interest, particularly
GRF, is wholly unexpected. The mutant a-type
S. cerevisiae yeast strain, in which such expression was
initially observed, is designated SB3-5B. This strain
is a legitimate a-strain, as it mates with alpha-strains
and does not secrete alpha-factor pheromone.

In one experiment, the expression
vector pTRP211 was transformed into an alpha-strain and
likewise transformed into the mutant a-strain SB3-5B.
GRF production in both strains peaked around thirty
hours at a level of approximately 1.5 mg/liter.

Polypeptide recovery is also enhanced by the
use of genetically altered or mutant yeast strains
having impaired proteolytic function as a host for
recombinant vectors in which the polypeptide-encoding
DNA sequence is linked to the alpha-factor leader
peptide-encoding sequence.

Data obtained with S. cerevisiae cells
(GG100-14D) transformed with the alpha-factor-based GRF
secretion vector, TRP213, indicated that some portion of
the recombinant GRF is proteolytically degraded. This
proteolysis is probably caused by endogenous secreted
proteases. The SB7-5D strain of S. cerevisiae bears the
SKI5 mutation, which renders the cells deficient in a
secreted protease (as yet uncharacterized). In an
attempt to limit proteolytic degradation, and thus increase the level of biologically active (undegraded) GRF, SB7-5D cells were transformed with TRP213 vector and the GRF production level, biological activity and protein characterization of the GRF analyzed.

SB7-5D transformants were grown in a shake flask utilizing normal shake flask growth conditions. The level of GRF produced, as measured by radioimmuno assay, was comparable to that obtained using the GG100-14D strain grown in a shake flask. In semi-purified form, the GRF has full biological activity ($ED_{50}$ about 40 pM). The biological activity of GG100-14D transformed with pTRP213 is about 10-fold lower when measured at the same stage of purification (shake flask grown). Protein characterization studies have shown that about 50% of the recombinant protein produced by SB7-5D comigrates with authentic GRF. The proteins eluted in two peaks off an HPLC column, while GG100-14D-produced proteins elute in three peaks. Thus, while it appears that production of GRF is substantially the same in both strains, recovery of biologically active GRF is significantly higher in the mutant SB7-5D strain in which proteolytic function is impaired.

Cultures of S. cerevisiae strains GG100-14D, SB3-5B and SB7-5D have been deposited at the ATCC under the terms of the Budapest Treaty on the International Recognition of the Deposit of Microorganisms for the Purposes of Patent Procedure and the Regulations promulgated thereunder. Samples of said cultures are and will be available to industrial property offices and other persons legally entitled to receive them under the terms of said Treaty and Regulations and in accordance with the patent laws and regulations of each country and international organization in which this application is filed or any patent based on this application is granted. The ATCC accession numbers of the strains are

0206783

as follows:

| | |
|---|---|
| GG100-14D | 20762 |
| SB3-5B | 20761 |
| SB7-5D | 20760 |

While the present invention has been described in some detail with respect to certain embodiments, modifications obvious to one with ordinary skill in the art may be made without departing from the scope of the invention.

0206783

CLAIMS:

1. An a-strain of Saccharomyces cerevisiae transformed with a plasmid having a DNA sequence that encodes a fusion polypeptide in which alpha-factor-leader peptide is linked to a heterologous polypeptide of interest, said a-strain expressing said fusion polypeptide.

2. A strain of Saccharomyces cerevisiae having a genetic deficiency which impairs its proteolytic function, said proteolytically-impaired strain being transformed with a plasmid having a DNA sequence that encodes a fusion polypeptide in which alpha-factor-leader peptide is linked to a heterologous polypeptide of interest, said strain expressing said fusion polypeptide.

3. A strain of Saccharomyces cerevisiae which is SB3-5B, SB7-5D or GG 100-14D.

4. A recombinant DNA vector for transformation into Saccharomyces cerevisiae comprising DNA sequences which render said vector compatible with its transformation and continued viability in S. cerevisiae yeasts, a DNA sequence encoding a fusion polypeptide, and a DNA sequence for promoting expression of said fusion polypeptide, said fusion polypeptide having the general formula $NH_2$- pre-pro-alpha-factor leader peptide-(alpha-factor peptide)$_n$-proteolytic processing site-heterologous polypeptide, where n = 1 or 2, preferably n=1 and wherein, if n=2, there is a proteolytic processing site of sequence lys-arg-glu-ala-glu-ala- between the alpha-factor peptides.

5. A recombinant DNA vector according to Claim 5 comprising a DNA sequence for pre-pro-alpha-factor leader peptide-(alpha-factor unit)$_n$-(lys-arg)-heterologous polypeptide, preferably wherein n=1.

6.   A recombinant DNA vector according to Claim 3 wherein said heterologous polypeptide is urokinase, IGF 1, GRF or a biologically active analog thereof

7.   A recombinant DNA vector according to Claim 6 wherein when said heterologous polypeptide is a GRF analog, it is selected from the group of polypeptide having the general formula:

$$H - R_1 - Ala - Asp - Ala - Ile - Phe - Thr -$$
$$R_8 - Ser - R_{10}- Arg - R_{12}- R_{13}- Leu -$$
$$Gly - Gln - Leu - R_{18}- Ala - Arg - Lys -$$
$$Leu - Leu - R_{24}- R_{25} -Ile - R_{27}- R_{28}-$$
$$Arg - Gln - Gln - Gly - Glu - R_{34}- Asn -$$
$$Glu - Glu - R_{38}- R_{39}- R_{40}- R_{41}- R_{42}-$$
$$R_{43}- R_{44}- OH$$

wherein $R_1$ is Tyr, Phe, Leu or His; $R_8$ is Asn or Ser; $R_{10}$ is Tyr or Phe; $R_{12}$ is Arg or Lys; $R_{13}$ is Ile or Val; $R_{18}$ is Ser or Tyr; $R_{24}$ is His or Gln; $R_{25}$ is Glu or Asp; $R_{27}$ is Ala, Ile, Leu or Val; $R_{28}$ is Ser or sn; $R_{34}$ is Ser, Ala or Arg; $R_{38}$ is Arg, Ser or Gln; $R_{39}$ is Arg or Gly; $R_{40}$ is Ala, Arg or Ser; $R_{41}$ is Arg or Lys; $R_{42}$ is Phe, Ala or Val; $R_{43}$ is Arg or Asn; and $R_{44}$ is a natural amino acid, excluding Cys or Met, wherein any or all of the residues between $R_{28}$ and $R_{44}$, inclusive, may be deleted.

8.   A recombinant DNA vector according to Claim 7 wherein said GRF analog is [Leu[27]]-hGRF)1-40)-OH or [Leu[27]]-hGRF(1-44)-OH.

9.  A strain of S. cerevisiae yeast transformed with the vector of any one of Claims 4 to 8, which transformed yeast strain expresses and secretes said heterologous polypeptide.

10.  A strain of S. cerevisiae yeast according to Claim 9 wherein said heterologous polypeptide is preferably secreted into the media at a level greater than or equal to 1 mg/1/0.D and/or wherein at least 20% possesses full biological activity.

11.  A strain of Saccharomyces cerevisiae yeast having a genetic deficiency which impairs its proteolytic function, said proteolytically-impaired strain being transformed with a vector according to Claim 4.

12.  A recombinant DNA sequence comprising an alpha-factor leader pre-segment-encoding sequence portion, a sequence portion encoding a GRF analog selected from [Leu$^{27}$]-hGRF(1-44)-OH and [Leu$^{27}$]-hGRF(1-40)-OH, and at least one proteolytic processing site-encoding sequence portion between said pre-segment-encoding sequence portion and said GRF analog-encoding sequence portion, said recombinant DNA sequence encoding said pre-segment and said GRF analog as parts of a single fusion polypeptide.

13.  A recombinant DNA sequence according to Claim 12 including one ot two alpha-factor-encoding sequence portions between said pre-segment-encoding sequence portion and said analog-encoding sequence portion, said alph-factor encoding sequence portions being bracketed by proteolytic processing site-encoding sequence portions, said proteolytic processing site-encoding sequences optionally coding for the dipeptide lys-arg and/or the dipeptide glu-ala.

14.  A method for the production of a desired protein which comprises culturing a strain of <u>S. cerevisiae</u> as defined in any one of Claims 1 to 3.

15.  A method for the production in <u>Saccharomyces cerevisiae</u> of biologically active polypeptide capable of stimulating the release of growth hormone by the pituitary which comprises:

culturing a host yeast cell transformed with a recombinant DNA vector encoding a polypeptide in such a manner that the host cell expresses the encoded polypeptide, wherein said recombinant DNA vector comprises

an alpha-factor leader pre-segment-encoding sequence portion,

a sequence portion encoding a GRF analog having the amino acid sequence:

$$H - R_1 - Ala - Asp - Ala - Ile - Phe - Thr -$$
$$R_8 - Ser - R_{10} - Arg - R_{12} - R_{13} - Leu -$$
$$Gly - Gln - Leu - R_{18} - Ala - Arg - Lys -$$
$$Leu - Leu - R_{24} - R_{25} - Ile - R_{27} - R_{28} -$$
$$Arg - Gln - Gln - Gly - Glu - R_{34} - Asn -$$
$$Glu - Glu - R_{38} - R_{39} - R_{40} - R_{41} - R_{42} -$$
$$R_{43} - R_{44} - OH$$

wherein $R_1$ is Tyr, Phe, Leu or His; $R_8$ is Asn or Ser; $R_{10}$ is Tyr or Phe; $R_{12}$ is Arg of Lys; $R_{13}$ is Ile or Val; $R_{18}$ is Ser or Tyr; $R_{24}$ is His or Gln; $R_{25}$ is Glu or Asp; $R_{27}$ is Ala, Ile, Leu or Val; $R_{28}$ is Ser or Asn; $R_{34}$ is Ser, Ala or Arg; $R_{38}$ is

Arg, Ser or Gln; $R_{39}$ is Arg or Gly; $R_{40}$ is Ala, Arg or Ser; $R_{41}$ is Arg or Lys; $R_{42}$ is Phe, Ala or Val; $R_{43}$ is Arg or Asn; and $R_{44}$ is a natural amino acid, excluding Cys or Met; wherein any or all of the residues between $R_{28}$ and $R_{44}$, inclusive, may be deleted, and

at least one proteolytic processing site-encoding sequence portion between said pre-segment-encoding sequence portion and said GRF analog-encoding sequence portion, wherein said recombinant DNA sequence encodes said pre-segment and said GRF analog as parts of a single fusion polypeptide.

said recombinant DNA vector optionally further comprising DNA sequences which render said vector compatible with its transformation and continued viability in S. cerevisiae yeasts, and

a DNA sequence for promoting expression of said fusion polypeptide.

16. A method according to Claim 15 wherein said fusion polypeptide has the general formula

$NH_2$-pre-pro-alpha-factor pre-segment peptide-(proteolytic processing site)$_1$-(alpha factor peptide)$_n$-(proteolytic processing site)$_2$-polypeptide, wherein n = 1 or 2, wherein the sequences of (proteolytic processing site)$_1$ and (proteolytic processing site)$_2$ are the same or different and wherein, if n=2, there is a third proteolytic processing site between the two alpha-factor peptides, the sequence of said third proteolytic processing site being different from the sequences of both of the other two processing sites or the same as the sequence of at least one of the other two processing sites.

17. A method according to Claim 15 wherein $R_1$ is Tyr and/or $R_8$ is Asn and/or $R_{10}$ is Tyr and/or

$R_{12}$ is Lys and/or $R_{13}$ is Val and/or $R_{18}$ is Ser and/or $R_{24}$ is Gln and/or $R_{25}$ is Asp and/or residues $R_{28}$ through $R_{44}$ are deleted.

18. A method according to Claim 16 wherein $R_1$ is Tyr, $R_8$ is Asn, $R_{10}$ is Tyr, $R_{12}$ is Lys, $R_{13}$ is Val, $R_{18}$ is Ser, $R_{24}$ is Gln, $R_{25}$ is Asp, $R_{27}$ is Leu, $R_{28}$ is Ser, $R_{34}$ is Ser, $R_{38}$ is Arg, $R_{39}$ is Gly, $R_{40}$ is Ala, $R_{41}$ is Arg, $R_{42}$ is Ala, $R_{43}$ is Arg, and $R_{44}$ is Leu.

19. A method according to Claim 16 wherein $R_1$ is Tyr, $R_8$ is Asn, $R_{10}$ is Tyr, $R_{12}$ is Lys, $R_{13}$ is Val, $R_{18}$ is Ser, $R_{24}$ is Gln, $R_{25}$ is Asp, and $R_{27}$ is leu.

20. A method according to Claim 16 wherein residues $R_{41}$ through $R_{44}$ are deleted and wherein $R_{27}$ is Leu.

21. The use in a recombinant DNA for transformation of S.cerevisiae of a sequence that encodes a fusion polypeptide in which alpha-factor-leader peptide is linked to a heterologous polypeptide of interest.

22. The use as claimed in Claim 21 wherein the polypeptide is as defined in any one of claims 6 to 8.

FIG.1.

RESTRICTION MAP OF ALPHA-MATING FACTOR (AMF) GENE

FIG.2.
YSV303

FIG 3
TRP 203

FIG.4
TRP 205

G3PdH TERMINATOR
HindIII — BamHI
Sal I
2μ ori
Xba I
EcoRI — PsT I
Hind III
Bgl II
TRP1
EcoRI
pBR322

FIG.5
TRP 209

α-FACTOR TERMINATOR
2μ ori — HindIII
EcoRI — Sal I — Xba I
PsT I
Xba I — BamHI
EcoRI
Xba I
Hind III
TRP1
PsT I
EcoRI
pBR322

0206783

3/5

FIG.6.
TRP 206

FIG.7.
TRP 210
TRP 211

FIG.8.
TRP 213

FIG.9.
TRP 215

FIG.10.
UROKINASE
EXPRESSION VECTOR